# EUROPEAN PATENT SPECIFICATION

(11) **EP 2 140 881 B1**
(45) Date of publication and mention of the grant of the patent: **17.04.2013**
(21) Application number: 09012818.2
(22) Date of filing: 14.12.2000
(51) Int. Cl.: A61K 39/395, A61K 38/49, A61K 38/18, A61K 38/19, A61P 25/00, A61P 9/10, C07K 16/28

(54) **Methods of treating central nervous system ischemic or hemorrhagic injury using anti alpha4 integrin antagonists**
Verfahren zur Behandlung von ischämischen oder hämorrhagischen Traumata des Zentralnervensystems unter Verwendung von Alpha4-Integrin-Antagonisten
Procédés de traitement de maladie hémorragique ou ischémique du système nerveux central, utilisant des antagonistes d'intégrine anti alpha 4

(30) Priority: 16.12.1999 US 171265 P
(43) Date of publication of application: 06.01.2010
(62) Divisional of application: 00984395.4
(73) Proprietor: Biogen Idec MA Inc., Cambridge, Massachusetts 02142 (US)
(72) Inventor: Relton, Jane, Belmont, MA 02478 (US); Lobb, Roy, Westwood, MA 02090 (US); Whalley, Eric, North Andover, MA 01845 (US); Adams, Steven P., Andover, MA 01810 (US)
(74) Representative: Vossius & Partner

(56) References cited:
- US-A- 5 260 210
- CLARK W M ET AL: "Reduction of central nervous system ischemic injury in rabbits using leukocyte adhesion antibody treatment." STROKE; A JOURNAL OF CEREBRAL CIRCULATION JUL 1991, vol. 22, no. 7, July 1991 (1991-07), pages 877-883, XP002556368 ISSN: 0039-2499
- YEDNOCK T A ET AL: "Prevention of experimental autoimmune encephalomyelitis by antibodies against alpha4/beta1 integrin" NATURE,MACMILLAN JOURNALS LTD. LONDON,GB, vol. 356, 5 March 1992 (1992-03-05), pages 63-66, XP002128375 ISSN: 0028-0836
- BAVBEK M ET AL: "Monoclonal antibodies against ICAM-1 and CD18 attenuate cerebral vasospasm after experimental subarachnoid hemorrhage in rabbits." STROKE; A JOURNAL OF CEREBRAL CIRCULATION SEP 1998, vol. 29, no. 9, September 1998 (1998-09), pages 1930-1935 ; di, XP002556369 ISSN: 0039-2499
- SOILU-HANNINEN M ET AL: "Therapy with antibody against leukocyte integrin VLA-4 (CD49d) is effective and safe in virus-facilitated experimental allergic encephalomyelitis." JOURNAL OF NEUROIMMUNOLOGY, vol. 72, no. 1, 1997, pages 95-105, XP000993010 ISSN: 0165-5728
- CLARK R S ET AL: "Antibodies against Mac-1 attenuate neutrophil accumulation after traumatic brain injury in rats." JOURNAL OF NEUROTRAUMA JUN 1996, vol. 13, no. 6, June 1996 (1996-06), pages 333-341, XP008115036 ISSN: 0897-7151
- KNOBLACH S M ET AL: "Early neuronal expression of tumor necrosis factor-alpha after experimental brain injury contributes to neurological impairment." JOURNAL OF NEUROIMMUNOLOGY 1 MAR 1999, vol. 95, no. 1-2, 1 March 1999 (1999-03-01), pages 115-125, XP002556370 ISSN: 0165-5728
- LABINAZ MARINO ET AL: "Infusion of an antialpha4 integrin antibody is associated with less neoadventitial formation after balloon injury of porcine coronary arteries." CANADIAN JOURNAL OF CARDIOLOGY, vol. 16, no. 2, February 2000 (2000-02), pages 187-196, XP000993009 ISSN: 0828-282X
- RELTON JANE K ET AL: "Inhibition of alpha4 integrin protects against transient focal cerebral ischemia in normotensive and hypertensive rats." STROKE, vol. 32, no. 1, January 2001 (2001-01), pages 199-205, XP000993039 ISSN: 0039-2499
- BECKER KYRA ET AL: "Antibody to the alpha4 integrin decreases infarct size in transient focal cerebral ischemia in rats." STROKE, vol. 32, no. 1, January 2001 (2001-01), pages 206-211, XP000993038 ISSN: 0039-2499

## Description

### Field of the Invention

The present invention relates generally to the treatment for acute Central Nervous System (CNS) injury. In particular, the invention relates to the use of antagonists of α4 integrins to treat ischemic CNS damage resulting from traumatic brain injury, or stroke. The α4 integrin antagonist chosen is an anti-α4 integrin antibody or an α4 integrin-binding fragment thereof and can be used as the sole therapeutic agent or in combination with other pharmacological agents.

### Background of the Invention

Acute central nervous system ("CNS") injuries encompass a wide variety of medical and traumatic insults to the brain and spinal cord. For example, stroke is the third leading cause of death in the developed world with one stroke occurring approximately every minute in the United States. Mortality rate is about 30% but more than 4 million stroke survivors are alive today, the majority of these individuals are left with varying degrees of disability. Clinical trials have yet to demonstrate therapeutic neuroprotection in ischemic stroke (i.e., stroke related to disruption of blood flow due to clot/thrombus formation) and spinal cord. Thrombolytic therapy (defined as use of an agent which causes dissolution or destruction of a thrombus) has many limitations, but it remains the only approved form of treatment for acute ischemic stroke. Current strategies being tested in the clinic to inhibit ischemic brain injury target excitotoxic mechanisms, nitric oxide associated neuronal damage, and ischemia associated neuronal cellular membrane damage. Pre-clinical research strategies are also targeting anti-apoptotic and anti-inflammatory mechanisms.

The pathophysiological responses to traumatic brain injury or TBI (e.g., brain injury caused by, among other things, head accidents and head wounds) are similar in many respects to those of stroke and similar approaches are being taken to develop therapeutics for the treatment of TBI. Whether or not a stroke is caused by ischemic or hemorrhagic mechanisms can be determined by a CAT scan or other clinical procedure and the mode of subsequent treatment will be dependent upon the results of this screening.

Cellular adhesion and trafficking across the vascular interface plays an essential role in both physiological and pathophysiological processes of acute brain injury. Of particular interest in the pathology of ischemic brain injury are polymorphonuclear leukocytes and T cells, which have been implicated in the development of brain damage after experimental stroke (Garcia et al 1994, Am. J. Pathol. 144:188; Becker et al, 1997 PNAS 94:10873). Cellular infiltration into the brain is thought to occur after brain injury and may contribute to disease progression. Thus, secondary brain damage (eg. hemorrhagic transformation, cerebral vasospasm) may also result from an acute brain injury in a subject. Spinal cord injury (SCI), like TBI occurs in a young healthy population but shares many pathological similarities to the changes occuring in the brain after a stroke. In light of such common mechanisms similar therapeutic approaches as those for stroke and TBI are being developed for the treatment of SCI.

Cell-cell or cell-matrix interactions are mediated through several families of cell adhesion molecules, one such family of which includes the integrins. Integrins are structurally and functionally related glycoproteins consisting of various alpha (alpha1, alpha 2, up to alpha 11 at present) and beta (beta 1 and beta 7) heterodimeric transmembrane receptor domains found in various combinations on virtually every mammalian cell type. (for reviews see: E. C. Butcher, Cell, 67, 1033 (1991); D. Cox et al., "The Pharmacology of the Integrins." Medicinal Research Rev. Vol. 195 (1994) and V. W. Engleman et al., "Cell Adhesion Integrins as Pharmaceutical Targets" in Ann, Revs.Medicinal Chemistry, Vol. 31, J. A. Bristol, Ed.; Acad. Press, NY, 1996, p. 191). Two alpha4 subunit containing integrins have been described and are designated alpha4betal (VLA-4) and alpha4beta7.

US 5,260,210 A discloses a blood brain barrier model designed to screen reagents useful to prevent or ameliorate brain inflammation.

An anti-CD 18 treatment in preserving neurologic function after central nervous system ischemia has been described in Clark et al. (Stroke (1991), vol. 22, no. 7, pages 877-883). The prevention of the accumulation of leucocytes in the central nervous system and the development of autoimmune encephalomyelitis has been described in Yednock et al. (Nature (1992), vol. 356, pages 63-66).

Previous experiments showed upregulation of the alpha4betal and alpha4beta7 counter receptor VCAM-1 in the brain after ischemic injury, but no data demonstrating a functional role in the disease were reported (Jander et al, 1996, J. NeuroImmunol. 70: 75). VLA-4 and alpha4beta7 are expressed on mononuclear leukocytes (see Lobb and Adams, 1994; J. C/in. Invest. 94:1722).

It would be useful to develop methods of antagonizing members of the integrin family in this context. Further, it would be useful to develop a therapeutic modality for stroke that is efficacious whether the injury is ischemic or hemorrhagic.

### Summary of the Invention

Until the present disclosure the pathological role of alpha4 subunit containing-integrins in CNS injury (e.g., cerebral ischemia) had not been defined. The present invention relates in part to the protective effect of inhibiting alpha4 subunit containing integrins in a rat model of focal cerebral ischemia.

The invention relates to the embodiments as defined in the claims.

Methods to treat CNS injury, such as stroke, using inhibitors of alpha4betal and/or alpha4beta7 are disclosed,

A method to treat acute CNS injury in a patient in need of such treatment, comprising administration of an alpha4 subunit containing integrin antagonist is also disclosed. Also disclosed is a method which includes further administering a pharmacological agent to the patient. Preferably, the acute CNS injury is stroke, traumatic brain injury or spinal cord injury. In some embodiments, the stroke is ischemic or hemorrhagic stroke.

The pharmacological agent may be a thrombolytic agent such as tissue plasminogen activator or urokinase or it may be a neuroprotective agent or anti-inflammatory agent. In certain aspects of the invention, the neuroprotective agent is an antagonist of a receptor, the receptor selected from the group consisting of: N-Methyl-D aspartate receptor (NMDA), α-amino-3-hydroxy-5-methyl-4-isoxazoleproprionic acid receptor (AMPA), glycine receptor, calcium channel receptor, bradykinin B2 receptor and sodium channel receptor. In other aspects of the invention, the anti-inflammatory agent is selected from the group consisting of interleukin-1, and tumor necrosis factor family members. The neuroprotective agent may also be an agonist of a receptor, the receptor selected from the group consisting of: the bradykinin B1 receptor, γ-amino butyric acid (GABA) receptor, and Adenosine A1 receptor.

The invention further relates to a method to treat secondary brain damage resulting from an ischemic insult in a patient in need of such treatment, comprising administration of an inhibitor of an α4 subunit containing integrin.

The inhibitor of the α4 subunit containing integrin according to the invention is an anti-α4 integrin antibody or an α4 integrin-binding fragment thereof.

Disclosed is a method to treat ischemic or hemorrhagic stroke using an inhibitor of the alpha4 subunit containing integrins alpha4betal or alpha4beta7 alone or together as the therapeutic agent, or alone or together in combination with other therapeutic agents.

It is an object of the present invention to provide a method to treat traumatic brain injury using an inhibitor of the alpha4 subunit containing integrin alpha4betal or alpha4beta7 alone or together as the therapeutic agent, or alone or together in combination with other therapeutic agents.

Further disclosed is a method to treat spinal cord injury using an inhibitor of the alpha 4 subunit containing integrins alpha4betal or alpha4 beta7 alone or together as the therapeutic agent, or alone or together in combination with other therapeutic agents.

It is a further object of this invention to provide a method to treat secondary brain damage occuring as a consequence of a primary ischemic insult (eg. Hemorrhagic transformation, cerebral vasospasm) using an inhibitior of the alpha4 subunit containing integrins alpha4betal or alpha4beta7 alone or together as the therapeutic agent, or alone or together in combination with other therapeutic agents.

### Brief description of drawings

Figure 1A depicts a graph of infarct volume (mm³) in cortical and subcortical regions of the brains of Sprague Dawley rats after treatment with hoe 140 (300ng/kg/min) and vehicle control.
Figure 1B depicts a graph of infarct volume (mm³) in cortical and subcortical regions of the brains of spontaneously hypertensive rats after treatment with hoe 140 (300 ng/kg/min) and vehicle control.
Figure 2A depicts a graph of infarct volume (mm³) in cortical and subcortical regions of the brains of Sprague Dawley rats after treatment with anti-rat-alpha4 antibody (TA-2, 2.5 mg/kg) and isotype control antibody.
Figure 2B depicts a graph of infarct volume (mm³) in cortical and subcortical regions of the brains of spontaneously hypertensive rats after treatment with anti-rat-alpha4 antibody (TA-2, 2.5 mg/kg) and isotype control antibody.

### Detailed Description of the Invention

### I. Definitions

In order to more clearly and concisely point out the subject matter of the claimed invention, the following definitions are provided for specific terms used in the following written description and appended claims.

The invention will now be described with reference to the following detailed description of which the following definitions are included:

The integrin very late antigen (VLA) superfamily is made up of structurally and functionally related glycoproteins consisting of (alpha and beta) heterodimeric, transmembrane receptor molecules found in various combinations on nearly every mammalian cell type. (for reviews see: E. C. Butcher, Cell, 67, 1033 (1991); D. Cox et al., "The Pharmacology of the Integrins." Medicinal Research Rev. (1994) and V. W. Engleman et al., 'Cell Adhesion Integrins as Pharmaceutical Targets.' in Ann. Report in Medicinal Chemistry, Vol. 31, J. A. Bristol, Ed.; Acad. Press, NY, 1996, p. 191). Integrins of the VLA family include (at present) VLA-1, -2, -3, -4, -5, -6, -9, and -11 in which each of the molecules comprise a β1 chain non- covalently bound to an alpha chain, (α1, α2, α3, α4, α5, α6 and the like), respectively.

Alpha 4 beta 1 (α1β1) integrin is a cell-surface receptor for VCAM-1, fibronectin and possibly other ligands (the latter ligands individually and collectively referred to as "alpha4 ligand(s)"). The term α4β1 integrin ("VLA-4" or "a4b1" or "a4b1 integrin", used interchangeably) herein thus refers to polypeptides which are capable of binding to VCAM-1 and members of the extracellular matrix proteins, most particularly fibronectin, or homologs or fragments thereof, although it will be appreciated by workers of ordinary skill in the art that other ligands for VLA-4 may exist and can be analyzed using conventional methods. Nevertheless, it is known that the alpha4 subunit will associate with other beta subunits besides betal so that we may define the term "alpha (I) 4 integrin" or "alpha (I) 4 subunit-containing integrin" as being those integrins whose alpha4 subunit associates with one or another of the beta subunits. Another example of an "alpha4" integrin besides VLA4 is alpha4beta7 (See Lobb and Adams, supra).

An integrin "antagonist" includes any compound that inhibits alpha4 subunit-containng integrins from binding with an integrin ligand and/or receptor. Anti-integrin antibody or antibody homolog-containing proteins (discussed below) as well as other molecules such as soluble forms of the ligand proteins for integrins are useful. Soluble forms of the ligand proteins for alpha4 subunit-containing integrins include soluble VCAM-1, VCAM-1 fusion proteins, or bifunctional VCAM-1/Ig fusion proteins. For example, a soluble form of an integrin ligand or a fragment thereof may be administered to bind to integrins, and preferably compete for an integrin binding site on cells, thereby leading to effects similar to the administration of antagonists such as anti-integrin (e.g., VLA-4) antibodies. In particular, soluble integrin mutants that bind ligand but do not elicit integrin-dependent signaling are disclosed.

Such integrins mutants can act as competitive inhibitors of wild type integrins protein and are considered "antagonists". Other antagonists disclosed are "small molecules", as defined below.

The agonists according to the invention are anti-α4 integrin antibodies or α4 integrin-binding fragments thereof.

Also disclosed are methods using molecules that antagonize the action of more than one alpha 4 subunit-containing integrin, such as small molecules or antibody homologs that antagonize both VLA-4 and alpha4 beta'7 or other combinations of alpha4 subunit-containing integrins. Also disclosed are methods using a combination of molecules such that the combination antagonizes the action of more than one integrin, such as methods using several small molecules or antibody homologs that in combination antagonize both VLA-4 and alpha4 beta7 or other combinations of alpha4 subunit-containing integrins.

As discussed herein, certain integrin antagonists can be fused or otherwise conjugated to, for instance, an antibody homolog such as an immunoglobulin or fragment thereof and are not limited to a particular type or structure of an integrin or ligand or other molecule. Thus, for purposes of the invention, any agent capable of forming a chimeric protein (as defined below) and capable of binding to integrin ligands and which effectively blocks or coats VLA- 4 (e.g., VLA-4) integrin is considered to be an equivalent of the antagonists used in the examples herein.

"Antibody homolog" includes intact antibodies consisting of immunoglobulin light and heavy chains linked via disulfide bonds. The term "antibody homolog" is also intended to encompass a protein comprising one or more polypeptides selected from immunoglobulin light chains, immunoglobulin heavy chains and antigen-binding fragments thereof which are capable of binding to one or more antigens (i.e., integrin or integrin ligand). The component polypeptides of an antibody homolog composed of more than one polypeptide may optionally be disulfide-bound or otherwise covalently crosslinked. Accordingly, therefore, "antibody homologs" include intact immunoglobulins of types IgA, IgG, IgE, IgD, IgM (as well as subtypes thereof), wherein the light chains of the immunoglobulin may be of types kappa or lambda. "Antibody homologs" also includes portions of intact antibodies that retain antigen-binding specificity, for example Fab fragments, Fab' fragments, F(ab')2 fragments, F(v) fragments, heavy and light chain monomers or dimers or mixtures thereof.

"Humanized antibody homolog" is an antibody homolog, produced by recombinant DNA technology, in which some or all of the amino acids of a human immunoglobulin light or heavy chain that are not required for antigen binding have been substituted for the corresponding amino acids from a nonhuman mammalian immunoglobulin light or heavy chain. A "human antibody homolog" is an antibody homolog in which all the amino acids of an immunoglobulin light or heavy chain (regardless of whether or not they are required for antigen binding) are derived from a human source.

As used herein, a "human antibody homolog" is an antibody homolog produced by recombinant DNA technology, in which all of the amino acids of an immunoglobulin light or heavy chain that are derived from a human source.

An integrin "agonist" includes any compound that activates the integrin ligand.

"Amino acid" is a monomeric unit of a peptide, polypeptide, or protein. There are twenty amino acids found in naturally occurring peptides, polypeptides and proteins, all of which are L-isomers. The term also includes analogs of the amino acids and D-isomers of the protein amino acids and their analogs.

"Covalently coupled" - means that the specified moieties of the invention (e.g., PEGylated alpha 4 integrin antagonist, immunoglobulin fragment/ alpha 4 integrin antagonist) are either directly covalently bonded to one another, or else are indirectly covalently joined to one another through an intervening moiety or moieties, such as a spacer moiety or moieties. The intervening moiety or moieties are called a "coupling group". The term "conjugated" is used interchangeably with "covalently coupled". In this regard a "spacer" refers to a moiety that may be inserted between an amino acid or other component of an alpha4 integrin antagonist or fragment and the remainder of the molecule. A spacer may provide separation between the amino acid or other component and the rest of the molecule so as to prevent the modification from interfering with protein function and/or make it easier for the amino acid or other component to link with another moiety.

"Expression control sequence"- a sequence of polynucleotides that controls and regulates expression of genes when operatively linked to those genes.

"Expression vector"- a polynucleotide, such as a DNA plasmid or phage (among other common examples) which allows expression of at least one gene when the expression vector is introduced into a host cell. The vector may, or may not, be able to replicate in a cell.

An "effective amount" of an agent of the invention is that amount which produces a result or exerts an influence on the particular condition being treated.

"Functional equivalent" of an amino acid residue is (i) an amino acid having similar reactive properties as the amino acid residue that was replaced by the functional equivalent; (ii) an amino acid of an antagonist of the invention, the amino acid having similar properties as the amino acid residue that was replaced by the functional equivalent; (iii) a non-amino acid molecule having similar properties as the amino acid residue that was replaced by the functional equivalent.

A first polynucleotide encoding a proteinaceous antagonist of the invention is "functionally equivalent" compared with a second polynucleotide encoding the antagonist protein if it satisfies at least one of the following conditions:
(a): the "functional equivalent" is a first polynucleotide that hybridizes to the second polynucleotide under standard hybridization conditions and/or is degenerate to the first polynucleotide sequence. Most preferably, it encodes a mutant protein having the activity of an integrin antagonist protein;
(b) the "functional equivalent" is a first polynucleotide that codes on expression for an amino acid sequence encoded by the second polynucleotide.

The integrin antagonists disclosed herein include, but are not limited to, the agents listed herein as well as their functional equivalents. As used herein, the term "functional equivalent" therefore refers to an integrin antagonist or a polynucleotide encoding the integrin antagonist that has the same or an improved beneficial effect on the recipient as the integrin antagonist of which it is deemed a functional equivalent. As will be appreciated by one of ordinary skill in the art, a functionally equivalent protein can be produced by recombinant techniques, e.g., by expressing a "functionally equivalent DNA". Accordingly, the instant invention embraces integrin proteins encoded by naturally-occurring DNAs, as well as by non-naturally-occurring DNAs which encode the same protein as encoded by the naturally-occurring DNA. Due to the degeneracy of the nucleotide coding sequences, other polynucleotides may be used to encode integrin protein. These include all, or portions of the above sequences which are altered by the substitution of different codons that encode the same amino acid residue within the sequence, thus producing a silent change. Such altered sequences are regarded as equivalents of these sequences. For example, Phe (F) is coded for by two codons, TTC or TTT, Tyr (Y) is coded for by TAC or TAT and His (H) is coded for by CAC or CAT. On the other hand, Trp (W) is coded for by a single codon, TGG. Accordingly, it will be appreciated that for a given DNA sequence encoding a particular integrin there will be many DNA degenerate sequences that will code for it. These degenerate DNA sequences are considered within the scope of this invention.

The term "chimeric" when referring to an antagonist of the invention, means that the antagonist is comprised of a linkage (chemical cross-linkage or covalent or other type) of two or more proteins having disparate structures and/or having disparate sources of origin. Thus, a chimeric alpha 4 integrin antagonist may include one moiety that is an alpha 4 integrin antagonist or fragment and another moiety that is not an alpha 4 integrin antagonist.

A species of 'chimeric' protein is a "fusion" or "fusion protein" which refers to a co-linear, covalent linkage of two or more proteins or fragments thereof via their individual peptide backbones, most preferably through genetic expression of a polynucleotide molecule encoding those proteins. Thus, preferred fusion proteins are chimeric proteins that include an alpha4 integrin antagonist or fragment covalently linked to a second moiety that is not an alpha 4 integrin antagonist. Preferred fusion proteins of the invention may include portions of intact antibodies that retain antigen-binding specificity, for example, Fab fragments. Fab' fragments, F(ab')2 fragments, F(v) fragments, heavy chain monomers or dimers, light chain monomers or dimers, dimers consisting of one heavy and one light chain, and the like.

The most preferred fusion proteins are chimeric and comprise an integrin antagonist moiety fused or otherwise linked to all or part of the hinge and constant regions of an immunoglobulin light chain, heavy chain, or both. Thus, this invention features a molecule which includes: (1) an integrin antagonist moiety, (2) a second peptide, e.g., one which increases solubility or in vivo life time of the integrin antagonist moiety, e.g., a member of the immunoglobulin super family or fragment or portion thereof, e.g., a portion or a fragment of IgG, e.g., the human IgGI heavy chain constant region, e.g., CH2, CH3, and hinge regions. Specifically, a "integrin antagonist/Ig fusion" is a protein comprising a biologically active integrin antagonist molecule of the invention (e.g. a soluble VLA-4 ligand, or a biologically active fragment thereof linked to an N-terminus of an immunoglobulin chain wherein a portion of the N-terminus of the immunoglobulin is replaced with the integrin antagonist. A species of integrin antagonist/Ig fusion is an "integrin /Fc fusion" which is a protein comprising an integrin antagonist of the invention linked to at least a part of the constant domain of an immunoglobulin. A preferred Fc fusion comprises a integrin antagonist of the invention linked to a fragment of an antibody containing the C terminal domain of the heavy immunoglobulin chains.

The term "fusion protein" also means an integrin antagonist chemically linked via a mono- or hetero- functional molecule to a second moiety that is not an integrin antagonist (resulting in a "chimeric" molecule) and is made de novo from purified protein as described below. Thus, one example of a chemically linked, as opposed to recombinantly linked, chimeric molecule that is a fusion protein may comprise: (1) an alpha 4 integrin subunit targeting moiety, e.g., a VCAM-1 moiety capable of binding to VLA-4) on the surface of VLA-4 bearing cells; (2) a second molecule which increases solubility or in vivo life time of the targeting moiety, e.g., a polyalkylene glycol polymer such as polyethylene glycol (PEG). The alpha4 targeting moiety can be any naturally occurring alpha4 ligand or fragment thereof, e.g., a VCAM-1 peptide or a similar conservatively substituted amino acid sequence.

"Heterologous promoter"- as used herein is a promoter which is not naturally associated with a gene or a purified nucleic acid.

"Homology"- as used herein is synonymous with the term "identity" and refers to the sequence similarity between two polypeptides, molecules, or between two nucleic acids. When a position in both of the two compared sequences is occupied by the same base or amino acid monomer subunit (for instance, if a position in each of the two DNA molecules is occupied by adenine, or a position in each of two polypeptides is occupied by a lysine), then the respective molecules are homologous at that position. The percentage homology between two sequences is a function of the number of matching or homologous positions shared by the two sequences divided by the number of positions compared x 100. For instance, if 6 of 10 of the positions in two sequences are matched or are homologous, then the two sequences are 60% homologous. By way of example, the DNA sequences CTGACT and CAGGTT share 50% homology (3 of the 6 total positions are matched). Generally, a comparison is made when two sequences are aligned to give maximum homology. Such alignment can be provided using, for instance, the method of Needleman et al., J. Mol Biol. 48: 443-453 (1970), implemented conveniently by computer programs described in more detail below. Homologous sequences share identical or similar amino acid residues, where similar residues are conservative substitutions for, or "allowed point mutations" of, corresponding amino acid residues in an aligned reference sequence. In this regard, a "conservative substitution" of a residue in a reference sequence are those substitutions that are physically or functionally similar to the corresponding reference residues, e.g., that have a similar size, shape, electric charge, chemical properties, including the ability to form covalent or hydrogen bonds, or the like. Particularly preferred conservative substitutions are those fulfilling the criteria defined for an "accepted point mutation" in Dayhoff et al., 5: Atlas of Protein Sequence and Structure, 5: Suppl. 3, chapter 22: 354-352, Nat. Biomed. Res. Foundation, Washington. D.C. (1978).

"Homology" and "identity" each refer to sequence similarity between two polypeptide sequences, with identity being a more strict comparison. Homology and identity can each be determined by comparing a position in each sequence which may be aligned for purposes of comparison. When a position in the compared sequence is occupied by the same amino acid residue, then the polypeptides can be referred to as identical at that position; when the equivalent site is occupied by the same amino acid (e.g., identical) or a similar amino acid (e.g., similar in steric and/or electronic nature), then the molecules can be refered to as homologous at that position. A percentage of homology or identity between sequences is a function of the number of matching or homologous positions shared by the sequences. An "unrelated" or "non-homologous" sequence shares less than 40 percent identity, though preferably less than 25 percent identity, with an AR sequence of the present invention.

Various alignment algorithms and/or programs may be used, including FASTA, BLAST or ENTREZ. FASTA and BLAST are available as a part of the GCG sequence analysis package (University of Wisconsin, Madison, Wis.), and can be used with, e.g., default settings. ENTREZ is available through the National Center for Biotechnology Information, National Library of Medicine, National Institutes of Health, Bethesda, Md. In one embodiment, the percent identity of two sequences can be determined by the GCG program with a gap weight of 1, e.g., each amino acid gap is weighted as if it were a single amino acid or nucleotide mismatch between the two sequences.

"Isolated" (used interchangeably with "substantially pure")- when applied to nucleic acid i.e., polynucleotide sequences that encode integrin antagonists, means an RNA or DNA polynucleotide, portion of genomic polynucleotide, cDNA or synthetic polynucleotide which, by virtue of its origin or manipulation: (i) is not associated with all of a polynucleotide with which it is associated in nature (e.g., is present in a host cell as an expression vector, or a portion thereof); or (ii) is linked to a nucleic acid or other chemical moiety other than that to which it is linked in nature; or (iii) does not occur in nature. By "isolated" it is further meant a polynucleotide sequence that is: (i) amplified *in vitro* by, for example, polymerase chain reaction (PCR); (ii) synthesized chemically; (iii) produced recombinantly by cloning; or (iv) purified, as by cleavage and gel separation. Thus, "substantially pure nucleic acid" is a nucleic acid which is not immediately contiguous with one or both of the coding sequences with which it is normally contiguous in the naturally occurring genome of the organism from which the nucleic acid is derived. Substantially pure DNA also includes a recombinant DNA which is part of a hybrid gene encoding additional integrin sequences.

Isolated" (used interchangeably with "substantially pure")- when applied to polypeptides means a polypeptide or a portion thereof which, by virtue of its origin or manipulation: (i) is present in a host cell as the expression product of a portion of an expression vector; or (ii) is linked to a protein or other chemical moiety other than that to which it is linked in nature; or (iii) does not occur in nature, for example, a protein that is chemically manipulated by appending, or adding at least one hydrophobic moiety to the protein so that the protein is in a form not found in nature.. By "isolated" it is further meant a protein that is: (i) synthesized chemically; or (ii) expressed in a host cell and purified away from associated and contaminating proteins. The term generally means a polypeptide that has been separated from other proteins and nucleic acids with which it naturally occurs. Preferably, the polypeptide is also separated from substances such as antibodies or gel matrices (polyacrylamide) which are used to purify it.

"Multivalent protein complex"- refers to a plurality of integrin antagonists (i.e., one or more). An anti-integrin antibody homolog or fragment may be cross-linked or bound to another antibody homolog or fragment. Each protein may be the same or different and each antibody homolog or fragment may be the same or different.

"Mutant" - any change in the genetic material of an organism, in particular any change (i.e., deletion, substitution, addition, or alteration) in a wild type polynucleotide sequence or any change in a wild type protein. The term "mutein" is used interchangeably with "mutant".

"Operatively linked"- a polynucleotide sequence (DNA, RNA) is operatively linked to an expression control sequence when the expression control sequence controls and regulates the transcription and translation of that polynucleotide sequence. The term "operatively linked" includes having an appropriate start signal (e.g., ATG) in front of the polynucleotide sequence to be expressed, and maintaining the correct reading frame to permit expression of the polynucleotide sequence under the control of the expression control sequence, and production of the desired polypeptide encoded by the polynucleotide sequence.

A "pharmacological agent", is defined as one or more compounds or molecules or other chemical entities administered to a subject (in addition to the antagonists of the invention) that affect the action of the antagonist. The term "pharmacological agent' as used herein refers to such an agent(s) that are administered during "combination therapy" where the antagonist of the invention is administered either prior to, after, or simultaneously with, administration of one or more pharmacological agents.

"Protein"- any polymer consisting essentially of any of the 20 amino acids. Although "polypeptide" is often used in reference to relatively large polypeptides, and "peptide" is often used in reference to small polypeptides, usage of these terms in the art overlaps and is varied. The term "protein" as used herein refers to peptides, proteins and polypeptides, unless otherwise noted.

The terms "peptide(s)", "protein(s)" and "polypeptide(s)" are used interchangeably herein. The terms "polynucleotide sequence" and "nucleotide sequence" are also used interchangeably herein

"Recombinant," as used herein, means that a protein is derived from recombinant, mammalian expression systems. Since integrin is not glycosylated nor contains disulfide bonds, it can be expressed in most prokaryotic and eukaryotic expression systems.

"Small molecule"- has the definition as in Section A2.

The phrase "surface amino acid" means any amino acid that is exposed to solvent when a protein is folded in its native form.

"Standard hybridization conditions"- salt and temperature conditions substantially equivalent to 0.5 X SSC to about 5 X SSC and 65 ° C for both hybridization and wash. The term "standard hybridization conditions" as used herein is therefore an operational definition and encompasses a range of hybridization conditions. Higher stringency conditions may, for example, include hybridizing with plaque screen buffer (0.2% polyvinylpyrrolidone, 0.2% Ficoll 400; 0.2% bovine serum albumin, 50 mM Tris-HCl (pH 7.5); 1 M NaCl; 0.1% sodium pyrophosphate; 1 % SDS); 10% dextran sulfate, and 100 µg/ml denatured, sonicated salmon sperm DNA at 65 ° C for 12-20 hours, and washing with 75 mM NaCl/7.5 mM sodium citrate (0.5 x SSC)/1% SDS at 65° C. Lower stringency conditions may, for example, include hybridizing with plaque screen buffer, 10% dextran sulfate and 110 µg/ml denatured, sonicated salmon sperm DNA at 55 ° C for 12-20 hours, and washing with 300 mM NaCl/30mM sodium citrate (2.0 X SSC)/1% SDS at 55 ° C. See also Current Protocols in Molecular Biology, John Wiley & Sons, Inc. New York, Sections 6.3.1-6.3.6, (1989).

A "therapeutic composition" as used herein is defined as comprising the antagonists of the invention and other biologically compatible ingredients. The therapeutic composition may contain excipients such as water, minerals and carriers such as protein.

An antagonist of the invention (and its therapeutic composition) is said to have "therapeutic efficacy," and an amount of the agent is said to be "therapeutically effective," if administration of that amount of the agent is sufficient to cause a clinically significant improvement in neurological recovery in a standard neurological test (Section IV) when administered to a subject (e.g., an animal model or human patient) after brain damage (eg cerebral ischemia or stroke).

Practice of the present invention will employ, unless indicated otherwise, conventional techniques of cell biology, cell culture, molecular biology, microbiology, recombinant DNA, protein chemistry, pharmacology and immunology, which are within the skill of the art. Such techniques are described in the literature.

### II. Description of the preferred Embodiments

### General

We have discovered that inhibition of the α4 integrins; α4β1 and/or α4β7 protects the brain against injury induced by acute insult. Using a rat model of stroke caused by temporary occlusion of the middle cerebral artery we have demonstrated a significant reduction in brain infarction after treatment with an alpha 4 integrin antagonists The relevance of animal models of stroke has been reviewed by Hunter et al (1996) Trends in Pharmacological Sciences 6:123. The rat model of reversible middle cerebral artery occlusion in both Sprague Dawley (SD) and Spontaneously Hypertensive Rats (SHRs) is widely viewed as the most clinically relevant of rodent stroke models. (Hunter et al (1996) Trends in Pharmacological Sciences 6:123).

### A. Integrin Antagonists

For the purposes of the invention an integrin antagonist can be an antagonist of any interaction between an integrin and its cognate ligand or receptor such that the normal function induced by ligand-receptor interactions is altered (i.e., prevented or slowed or otherwise modified). One preferred embodiment of an integrin antagonist is an antagonist of interactions of alpha4 integrins with their ligand, such as the VCAM-1/VLA-4 interaction. This is an agent, e.g., a polypeptide or other molecule, which can inhibit or block VCAM-1 and/or VLA-4-mediated binding or which can otherwise modulate VCAM-1 and/or VLA-4 function, e.g., by inhibiting or blocking VLA-4-ligand mediated VLA-4 signal transduction or VCAM-1-ligand mediated VCAM-1 signal transduction and which is effective in the treatment of acute brain injury, preferably in the same manner as are anti-VLA-4 antibodies.

An antagonist of the VCAM-1/VLA-4 interaction is an agent which has one or more of the following properties: (1) it coats, or binds to, VLA-4 on the surface of a VLA-4 bearing cell (e.g., an endothelial cell) with sufficient specificity to inhibit a VLA-4-ligand/VLA-4 interaction, e.g., the VCAM-1/VLA-4 interaction; (2) it coats, or binds to, VLA-4 on the surface of a VLA-4 bearing cell (i.e., a lymphocyte) with sufficient specificity to modify, and preferably to inhibit, transduction of a VLA-4-mediated signal e.g., VLA-4/VCAM-1-mediated signaling; (3) it coats, or binds to, a VLA-4-ligand, (e.g., VCAM- 1) on endothelial cells with sufficient specificity to inhibit the VLA- 4 NCAM-1 interaction; (4) it coats, or binds to, a VLA-4-ligand (e.g., VCAM-1) with sufficient specificity to modify, and preferably to inhibit, transduction of VLA-4-ligand mediated VLA-4 signaling, e.g., VCAM-1-mediated VLA-4 signaling. In preferred embodiments the antagonist has one or both of properties 1 and 2. In other preferred embodiments the antagonist has one or both of properties 3 and 4. Moreover, more than one antagonist can be administered to a patient, e.g., an agent which binds to VLA-4 can be combined with an agent which binds to VCAM-1.

As discussed herein, the antagonists disclosed are not limited to a particular type or structure of molecule so that, for purposes of the invention, any agent capable of binding to alpha4 integrins (e.g., VLA-4) on the surface of cells or to an alpha4 ligand such as VCAM-1 on the surface of alpha4 ligand-bearing cells) and which effectively blocks or coats alpha 4 integrin (e.g., VLA-4) or alpha 4 ligand (e.g., VCAM-1), called an "alpha4 integrin binding agent" and "alpha4 integrin ligand binding agent" respectively), is considered to be an equivalent of the antagonists used in the examples herein.

For example, antibodies or antibody homologs (discussed below) as well as soluble forms of the natural binding proteins for VLA-4 and VCAM-1 are useful. Soluble forms of the natural binding proteins for VLA-4 include soluble VCAM-1 peptides, SCAM-1 fusion proteins, bifunctional VCAM-1/Ig fusion proteins (e.g. "chimeric" molecules, discussed above), fibronectin, fibronectin having an alternatively spliced non-type III connecting segment, and fibronectin peptides containing the amino acid sequence EILDV or a similar conservatively substituted amino acid sequence. Soluble forms of the natural binding proteins for VCAM-1 include soluble VLA-4 peptides, VLA-4 fusion proteins, bifunctional VLA-4/Ig fusion proteins and the like. As used herein, a "soluble VLA-4 peptide" or a "soluble VCAM-1 peptide" is an VLA-4 or VCAM-1 polypeptide incapable of anchoring itself in a membrane. Such soluble polypeptides include, for example, VLA-4 and VCAM polypeptides that lack a sufficient portion of their membrane spanning domain to anchor the polypeptide or are modified such that the membrane spanning domain is non-functional. These binding agents can act by competing with the cell-surface binding protein for VLA-4 or by otherwise altering VLA-4 function. For example, a soluble form of VCAM-1 (see, e.g., Osborn et al. 1989, Cell, 59: 1203-1211) or a fragment thereof may be administered to bind to VLA-4, and preferably compete for a VLA-4 binding site on VCAM-1-bearing cells, thereby leading to effects similar to the administration of antagonists such as small molecules or anti-VLA-4 antibodies.

### 1. Anti-Integrin Antibody Homologs

In other preferred embodiments, the antagonists used in the method of the invention to bind to, including block or coat, cell-surface alpha4 integrin (such as VLA-4 or alpha4 beta7) and/or cell surface ligand for alpha 4 integrin (such as VCAM-1) is an anti-VLA-4 and/or anti-VCAM-1 monoclonal antibody or antibody homolog, as defined previously. Preferred antibodies and homologs for treatment, in particular for human treatment, include human antibody homologs, humanized antibody homologs, chimeric antibody homologs, Fab, Fab', F(ab')2 and F(v) antibody fragments, and monomers or dimers of antibody heavy or light chains or mixtures thereof. Monoclonal antibodies against VLA-4 are a preferred binding agent in the method of the invention.

### 2. Small Molecule Integrin Antagonists

The term " small molecule" integrin antagonist refers to chemical agents (i.e., organic molecules) capable of disrupting the integrin/integrin ligand interaction by, for instance, blocking VLA-4/VCAM interactions by binding VLA-4 on the surface of cells or binding VCAM-1 on the surface of cells. Such small molecules may also bind respective VLA-4 and VCAM-1 receptors. VLA-4 and VCAM-1 small molecule inhibitors may themselves be peptides, semi-peptidic compounds or non-peptidic compounds, such as small organic molecules that are antagonists of the VCAM-1/VLA-4 interaction. A "small molecule", as defined herein, is not intended to encompass an antibody or antibody homolog. The molecular weight of exemplary small molecules is generally less than 1000.

For instance, small molecules such as oligosaccharides that mimic the binding domain of a VLA-4 ligand and fit the receptor domain of VLA-4 may be employed. (See, J.J. Devlin et al., 1990, Science 249: 400-406 (1990), J.K. Scott and G.P. Smith, 1990, Science 249: 386-390, and U.S. Patent 4,833,092 (Geysen) ). Conversely, small molecules that mimic the binding domain of a VCAM-1 ligand and fit the receptor domain of VCAM-1 may be employed.

Examples of other small molecules can be found in Komoriya et al. ("The Minimal Essential Sequence for a Major Cell Type-Specific Adhesion Site (CS1) Within the Alternatively Spliced Type III Connecting Segment Domain of Fibronectin Is Leucine-Aspartic Acid-Valine", J. Biol. Chem., 266 (23), pp. 15075-79 (1991)). They identified the minimum active amino acid sequence necessary to bind VLA-4 and synthesized a variety of overlapping peptides based on the amino acid sequence of the CS-1 region (the VLA-4 binding domain) of a particular species of fibronectin. They identified an 8-amino acid peptide, Glu-Ile-Ixu-Asp-Val-Pro-Ser-Thr, as well as two smaller overlapping pentapeptides, Glu-Ile-Leu-Asp-Val and Leu-Asp-Val-Pro-Ser, that possessed inhibitory activity against fibronectin-dependent cell adhesion. Certain larger peptides containing the LDV sequence were subsequently shown to be active in vivo (T. A. Ferguson et al., "Two Integrin Binding Peptides Abrogate T-cell-Mediated Immune Responses In Vivo", Proc. Natl. Acad. Sci. USA, 88, pp. 8072-76 (1991); and S. M. Wahl et al., "Synthetic Fibronectin Peptides Suppress Arthritis in Rats by Interrupting Leukocyte Adhesion and Recruitment", J. Clin. Invest., 94, pp. 655-62 (1994)). A cyclic pentapeptide, Arg-Cys-Asp-TPro-Cys (wherein TPro denotes 4-thioproline), which can inhibit both VLA-4 and VLA-5 adhesion to fibronectin has also been described. (See, e.g., D.M. Nowlin et al. "A Novel Cyclic Pentapeptide Inhibits Alpha4Betal Integrin-mediated Cell Adhesion", J. Biol. Chem., 268(27), pp. 20352-59 (1993); and PCT publication PCT/US91/04862). This pentapeptide was based on the tripeptide sequence Arg-Gly-Asp from fibronectin which had been known as a common motif in the recognition site for several extracellular-matrix proteins. Examples of other VLA-4 inhibitors have been reported. for example, in Adams et al. "Cell Adhesion Inhibitors", PCT US97/13013, describing linear peptidyl compounds containing beta-amino acids which have cell adhesion inhibitory activity. International patent applications WO 94/15958 and WO 92/00995 describe cyclic peptide and peptidomimetic compounds with cell adhesion inhibitory activity. International patent applications WO 93/08823 and WO 92/08464 describe guanidinyl-, urea- and thiourea-containing cell adhesion inhibitory compounds. United States Patent No. 5,260,277 describes guanidinyl cell adhesion modulation compounds. Other peptidyl antagonists of VLA-4 have been described in D. Y. Jackson et al., "Potent (α4β1 peptide antagonists as potential anti-inflammatory agents', J. Med. Chem., 40.3359 (1997); H. Shroff et al., 'Small peptide inhibitors of α4β7 mediated MadCAM-1 adhesion to lymphocytes", Bio. Med. Chem. Lett., 1 2495 (1996); U.S Patent 5,510,332, PCT Publications WO 98/53814, WO97/03094, WO97/02289, WO96140781, WO96/22966,WO96/20216, WO96/01644, WO96106108, and WO95/15973, and others.

Such small molecule agents may be produced by synthesizing a plurality of peptides (e.g., 5 to 20 amino acids in length), semi-peptidic compounds or non-peptidic, organic compounds, and then screening those compounds for their ability to inhibit the VLA-4/VCAM interaction. See generally U.S. Patent No. 4,833,093, Scott and Smith, "Searching for Peptide Ligands with an Epitope Library", Science, 249, pp. 386-90 (1990), and Devlin et al., "Random Peptide Libraries: A Source of Specific Protein Binding Molecules", Science, 249, pp. 40407 (1990).

### B. Methods of Making Anti-Integrin Antibody Homologs

The preferred integrin antagonists contemplated herein can be expressed from intact or truncated genomic or cDNA or from synthetic DNAs in prokaryotic or eukaryotic host cells. The dimeric proteins can be isolated from the culture media and/or refolded and dimerized in vitro to form biologically active compositions. Heterodimers can be formed in vitro by combining separate, distinct polypeptide chains. Alternatively, heterodimers can be formed in a single cell by co-expressing nucleic acids encoding separate, distinct polypeptide chains. See, for example, WO93/09229, or U.S. Pat. No. 5,411,941, for several exemplary recombinant heterodimer protein production protocols. Currently preferred host cells include, without limitation, prokaryotes including E. coli, or eukaryotes including yeast, Saccharomyces, insect cells, or mammalian cells, such as CHO, COS or BSC cells. One of ordinary skill in the art will appreciate that other host cells can be used to advantage. Detailed descriptions of the proteins useful in the practice of this invention, including how to make, use and test them for chondrogenic activity, are disclosed in numerous publications, including U.S. Pat. Nos. 5,266,683 and 5,011,6911.

The technology for producing monoclonal antibody homologs is well known. Briefly, an immortal cell line (typically myeloma cells) is fused to lymphocytes (typically splenocytes) from a mammal immunized with whole cells expressing a given antigen, e.g., VLA-4, and the culture supernatants of the resulting hybridoma cells are screened for antibodies against the antigen. See, generally, Kohler et at., 1975, Nature, 265: 295-297. Immunization may be accomplished using standard procedures.. The unit dose and immunization regimen depend on the species of mammal immunized, its immune status, the body weight of the mammal, etc. Typically, the immunized mammals are bled and the serum from each blood sample is assayed for particular antibodies using appropriate screening assays. For example, anti-VLA-4 antibodies may be identified by immunoprecipitation of 125I-labeled cell lysates from VLA-4-expressing cells. (See, Sanchez-Madrid et al. 1986, Eur. J. Immunol., 16: 1343-1349 and Hemler et al. 1987, J. Biol. Chem., 262, 11478-11485). Anti-VLA-4 antibodies may also be identified by flow cytometry, e.g., by measuring fluorescent staining of Ramos cells incubated with an antibody believed to recognize VLA-4 (see, Elices et al.. 1990 Cell, 60: 577-584). The lymphocytes used in the production of hybridoma cells typically are isolated from immunized mammals whose sera have already tested positive for the presence of anti-VLA-4 antibodies using such screening assays.

Typically, the immortal cell line (e.g., a myeloma cell line) is derived from the same mammalian species as the lymphocytes. Preferred immortal cell lines are mouse myeloma cell lines that are sensitive to culture medium containing hypoxanthine, aminopterin and thymidine ("HAT medium"). Typically, HAT-sensitive mouse myeloma cells are fused to mouse splenocytes using 1500 molecular weight polyethylene glycol ("PEG 1500"). Hybridoma cells resulting from the fusion are then selected using HAT medium, which kills unfused and unproductively fused myeloma cells (unfused splenocytes die after several days because they are not transformed). Hybridomas producing a desired antibody are detected by screening the hybridoma culture supernatants. For example, hybridomas prepared to produce anti-VLA-4 antibodies may be screened by testing the hybridoma culture supernatant for secreted antibodies having the ability to bind to a recombinant alpha4-subunit-expressing cell line (see, Elices et al., supra).

To produce anti-VLA-4 antibody homolog that are intact immunoglobulins, hybridoma cells that tested positive in such screening assays were cultured in a nutrient medium under conditions and for a time sufficient to allow the hybridoma cells to secrete the monoclonal antibodies into the culture medium. Tissue culture techniques and culture media suitable for hybridoma cells are well known. The conditioned hybridoma culture supernatant may be collected and the anti-VLA4 antibodies optionally further purified by well-known methods.

Alternatively, the desired antibody may be produced by injecting the hybridoma cells into the peritoneal cavity of an unimmunized mouse. The hybridoma cells proliferate in the peritoneal cavity, secreting the antibody which accumulates as ascites fluid. The antibody may be harvested by withdrawing the ascites fluid from the peritoneal cavity with a syringe.

Several mouse anti-VLA-4 monoclonal antibodies have been previously described. See, e.g., Sanchez-Madrid et al., 1986, supra; Hemler et al., 1987, supra; Pulido et al., 1991. J. Biol. Chem., 266 (16), 10241-10245): Issekutz and Wykretowicz, 1991, J. Immunol., 147: 109 (TA-2 mab). These anti-VLA-4 monoclonal antibodies and other anti-VLA-4 antibodies (e.g., U.S. Patent 5,888,507- Biogen, Inc. and references cited therein) capable of recognizing the alpha and/or beta chain of VLA-4 will be useful in the methods of treatment according to the present invention. AntiVLA-4 antibodies that will recognize the VLA-4 alpha4 chain epitopes involved in binding to VCAM-1 and fibronectin ligands (i.e., antibodies which can bind to VLA-4 at a site involved in ligand recognition and block VCAM-1 and fibronectin binding) are preferred. Such antibodies have been defined as B epitope-specific antibodies (B1 or B2) (Pulido et al., 1991, supra) and are also anti-VLA-4 antibodies according to the present invention.

Fully human monoclonal antibody homologs against VLA-4 are another preferred binding agent which may block or coat VLA-4 ligands in the method of the invention. In their intact form these may be prepared using in vitro-primed human splenocytes, as described by Boerner et al., 1991, J. Immunol., 147, 86-95. Alternatively, they may be prepared by repertoire cloning as described by Persson et al., 1991, Proc. Nat. Acad. Sci. USA, 88: 2432-2436 or by Huang and Stollar, 1991. J. Immunol. Methods 141, 227-236. U.S. Patent 5,798,230 (Aug. 25, 1998, "Process for the preparation of human monoclonal antibodies and their use") who describe preparation of human monoclonal antibodies from human B cells. According to this process, human antibody-producing B cells are immortalized by infection with an Epstein-Barr virus, or a derivative thereof, that expresses Epstein-Barr virus nuclear antigen 2 (EBNA2). EBNA2 function, which is required for immortalization, is subsequently shut off, which results in an increase in antibody production.

In yet another method for producing fully human antibodies, United States Patent 5,789,650 (Aug. 4, 1998, " Transgenic non-human animals for producing heterologous antibodies") describes transgenic non-human animals capable of producing heterologous antibodies and transgenic non-human animals having inactivated endogenous immunoglobulin genes. Endogenous immunoglobulin genes are suppressed by antisense polynucleotides and/or by antiserum directed against endogenous immunoglobulins. Heterologous antibodies are encoded by immunoglobulin genes not normally found in the genome of that species of non-human animal. One or more transgenes containing sequences of unrearranged heterologous human immunoglobulin heavy chains are introduced into a non-human animal thereby forming a transgenic animal capable of functionally rearranging transgenic immunoglobulin sequences and producing a repertoire of antibodies of various isotypes encoded by human immunoglobulin genes. Such heterologous human antibodies are produced in B-cells which are thereafter immortalized, e.g., by fusing with an immortalizing cell line such as a myeloma or by manipulating such B-cells by other techniques to perpetuate a cell line capable of producing a monoclonal heterologous, fully human antibody homolog.

Large nonimmunized human phage display libraries may also be used to isolate high affinity antibodies that can be developed as human therapeutics using standard phage technology (Vaughan et al, 1996).

Yet another preferred binding agent which may block or coat integrin ligands in the method of the invention is a humanized recombinant antibody homolog having anti-integrin specificity. Following the early methods for the preparation of true "chimeric antibodies" (where the entire constant and entire variable regions are derived from different sources), a new approach was described in EP 0239400 (Winter et al.) whereby antibodies are altered by substitution (within a given variable region) of their complementarity determining regions (CDRs) for one species with those from another. This process may be used, for example, to substitute the CDRs from human heavy and light chain Ig variable region domains with alternative CDRs from murine variable region domains. These altered Ig variable regions may subsequently be combined with human Ig constant regions to created antibodies which are totally human in composition except for the substituted murine CDRs. Such CDR-substituted antibodies would be predicted to be less likely to elicit an immune response in humans compared to true chimeric antibodies because the CDR-substituted antibodies contain considerably less non-human components. The process for humanizing monoclonal antibodies via CDR "grafting" has been termed "reshaping". (Riechmann et al., 1988, Nature 332, 323-327; Verhoeyen et al., 1988, Science 239, 1534-1536).

Typically, complementarity determining regions (CDRs) of a murine antibody are transplanted onto the corresponding regions in a human antibody, since it is the CDRs (three in antibody heavy chains, three in light chains) that are the regions of the mouse antibody which bind to a specific antigen. Transplantation of CDRs is achieved by genetic engineering whereby CDR DNA sequences are determined by cloning of murine heavy and light chain variable (V) region gene segments, and are then transferred to corresponding human V regions by site directed mutagenesis. In the final stage of the process, human constant region gene segments of the desired isotype (usually gamma I for CH and kappa for CL) are added and the humanized heavy and light chain genes are co-expressed in mammalian cells to produce soluble humanized antibody.

The transfer of these CDRs to a human antibody confers on this antibody the antigen binding properties of the original murine antibody. The six CDRs in the murine antibody are mounted structurally on a V region "framework" region. The reason that CDR-grafting is successful is that framework regions between mouse and human antibodies may have very similar 3-D structures with similar points of attachment for CDRS, such that CDRs can be interchanged. Such humanized antibody homologs may be prepared, as exemplified in Jones et al., 1986, Nature 321, 522-525; Riechmann, 1988, Nature 332, 323-327; Queen et al., 1989, Proc. Nat. Acad. Sci. USA 86, 10029; and Orlandi et al., 1989, Proc. Nat. Acad. Sci. USA 86, 3833.

Nonetheless, certain amino acids within framework regions are thought to interact with CDRs and to influence overall antigen binding affinity. The direct transfer of CDRs from a murine antibody to produce a recombinant humanized antibody without any modifications of the human V region frameworks often results in a partial or complete loss of binding affinity. In a number of cases, it appears to be critical to alter residues in the framework regions of the acceptor antibody in order to obtain binding activity.

Queen et al., 1989 (supra) and WO 90/07861 (Protein Design Labs) have described the preparation of a humanized antibody that contains modified residues in the framework regions of the acceptor antibody by combining the CDRs of a murine MAb (anti-Tac) with human immunoglobulin framework and constant regions. They have demonstrated one solution to the problem of the loss of binding affinity that often results from direct CDR transfer without any modifications of the human V region framework residues; their solution involves two key steps. First, the human V framework regions are chosen by computer analysts for optimal protein sequence homology to the V region framework of the original murine antibody, in this case, the anti-Tac MAb. In the second step, the tertiary structure of the murine V region is modelled by computer in order to visualize framework amino acid residues which arc likely to interact with the murine CDRs and these murine amino acid residues are then superimposed on the homologous human framework. See also U.S. Patents 5,693,762; 5,693,761; 5,585,089; and 5,530,101 (Protein Design Labs).

One may use a different approach (Tempest et al.. 1991. Biotechnology 9, 266-271) and utilize, as standard, the V region frameworks derived from NEWM and REI heavy and light chains respectively for CDR-grafting without radical introduction of mouse residues. An advantage of using the Tempest et al., approach to construct NEWM and REI based humanized antibodies is that the 3dimensional structures of NEWM and REI variable regions are known from x-ray crystallography and thus specific interactions between CDRs and V region framework residues can be modeled.

Regardless of the approach taken, the examples of the initial humanized antibody homologs prepared to date have shown that it is not a straightforward process. However, even acknowledging that such framework changes may be necessary, it is not possible to predict, on the basis of the available prior art, which, if any, framework residues will need to be altered to obtain functional humanized recombinant antibodies of the desired specificity. Results thus far indicate that changes necessary to preserve specificity and/or affinity are for the most part unique to a given antibody and cannot be predicted based on the humanization of a different antibody.

Certain alpha4 subunit-containing integrin antagonists useful in the present invention include chimeric and humanized recombinant antibody homologs (i.e., intact immunoglobulins and portions thereof) with B epitope specificity that have been prepared and are described in U.S. Patent 5,932,214(mab HP1/2). The starting material for the preparation of chimeric (mouse Variable - human Constant) and humanized anti-integrin antibody homologs may be a murine monoclonal anti-intearin antibody as previously described, a monoclonal anti-integrin antibody commercially available (e.g., HP2/1, Amae International, Inc., Westbrook, Maine), or a monoclonal anti-integrin antibody prepared in accordance with the teaching herein. Other preferred humanized anti-VLA4 antibody homolog are described by Athena Neurosciences, Inc. in PCT/US95/01219 (27 July 1995) and U.S. Patent 5,840,299.

These humanized anti-VLA-4 antibodies comprise a humanized light chain and a humanized heavy chain. The humanized light chain comprises three complementarity determining regions (CDRI, CDR2 and CDR3) having amino acid sequences from the corresponding complementarity determining regions of a mouse 21- 6 immunoglobulin light chain, and a variable region framework from a human kappa light chain variable region framework sequence except in at least position the amino acid position is occupied by the same amino acid present in the equivalent position of the mouse 21.6 immunoglobulin light chain variable region framework. The humanized heavy chain comprises three complementarity determining regions (CDR1, CDR2 and CDR3) having amino acid sequences from the corresponding complementarity determining regions of a mouse 21-6 immunoglobulin heavy chain, and a variable region framework from a human heavy chain variable region framework sequence except in at least one position the amino acid position is occupied by the same amino acid present in the equivalent position of the mouse 21-6 immunoglobulin heavy chain variable region framework.

### C. Production of Fragments and Analogs

Fragments of an isolated alpha4 integrin antibody (e.g., fragments of antibody homologs described herein) can also be produced efficiently by recombinant methods, by proteolytic digestion, or by chemical synthesis using methods known to those of skill in the art. In recombinant methods, internal or terminal fragments of a polypeptide can be generated by removing one or more nucleotides from one end (for a terminal fragment) or both ends (for an internal fragment) of a DNA sequence which encodes for the isolated hedgehog polypeptide. Expression of the mutagenized DNA produces polypeptide fragments. Digestion with "end nibbling" endonucleases can also generate DNAs which encode an array of fragments. DNAs which encode fragments of a protein can also be generated by random shearing, restriction digestion, or a combination or both. Protein fragments can be generated directly from intact proteins. Peptides can be cleaved specifically by proteolytic enzymes, including, but not limited to plasmin, thrombin, trypsin, chymotrypsin, or pepsin. Each of these enzymes is specific for the type of peptide bond it attacks. Trypsin catalyzes the hydrolysis of peptide bonds in which the carbonyl group is from a basic amino acid, usually arginine or lysine. Pepsin and chymotrypsin catalyse the hydrolysis of peptide bonds from aromatic amino acids, such as tryptophan, tyrosine, and phenylalanine. Alternative sets of cleaved protein fragments are generated by preventing cleavage at a site which is suceptible to a proteolytic enzyme. For instance, reaction of the ε-amino acid group of lysine with ethyltrifluorothioacetate in mildly basic solution yields blocked amino acid residues whose adjacent peptide bond is no longer susceptible to hydrolysis by trypsin. Proteins can be modified to create peptide linkages that are susceptible to proteolytic enzymes. For instance, alkylation of cysteine residues with β-haloethylamines yields peptide linkages that are hydrolyzed by trypsin (Lindley, (1956) Nature 178, 647). In addition, chemical reagents that cleave peptide chains at specific residues can be used. For example, cyanogen bromide cleaves peptides at methionine residues (Gross and Witkip, (1961) J. Am. Chem. Soc. 83, 1510). Thus, by treating proteins with various combinations of modifiers, proteolytic enzymes and/or chemical reagents, the proteins may be divided into fragments of a desired length with no overlap of the fragments, or divided into overlapping fragments of a desired length.

Fragments can also be synthesized chemically using techniques known in the art such as the Merrifield solid phase F moc or t-Boc chemistry. Merrifield, Recent Progress in Hormone Research 23: 451 (1967):

Examples of prior art methods which allow production and testing of fragments and analogs are discussed below. These, or analogous methods may be used to make and screen fragments and analogs of an isolated alpha4 integrin antagonist which can be shown to have biological activity. An exemplary method to test whether fragments and analogs of alpha 4 subunit containing integrin antagonists have biological activity is found in Section IV and the Examples.

### D. Production of Altered DNA and Peptide Sequences: Random Methods

Amino acid sequence variants of a protein can be prepared by random mutagenesis of DNA which encodes the protein or a particular portion thereof. Useful methods include PCR mutagenesis and saturation mutagenesis. A library of random amino acid sequence variants can also be generated by the synthesis of a set of degenerate oligonucleotide sequences. Methods of generating amino acid sequence variants of a given protein using altered DNA and peptides are well-known in the art. The following examples of such methods are not intended to limit the scope of the present invention, but merely serve to illustrate representative techniques. Persons having ordinary skill in the art will recognize that other methods are also useful in this regard, such as PCR Mutagenesis, Saturation Mutagenesis and degenerate oligonucleotide mutagenesis, as described in the below-cited references.
PCR Mutagenesis: See, for example Leung et al., (1989) Technique 1, 11 - 15. Saturation Mutagenesis: One method is described generally in Mayers et al., (1989) Science 229, 242.
Degenerate Oligonucleotide Mutagenesis: See for example Harang, S.A., (1983) Tetrahedron 39, 3; Itakura et al., (1984) Ann. Rev. Biochem. 53, 323 and Itakura et al. Recombinant DNA, Proc. 3rd Cleveland Symposium on Macromolecules, pp. 273-289 (A.G. Walton, ed.), Elsevier, Amsterdam, 1981.

### E. Production of Altered DNA and Peptide Sequences: Directed Methods

Non-random, or directed, mutagenesis provides specific sequences or mutations in specific portions of a polynucleotide sequence that encodes an isolated polypeptide, to provide variants which include deletions, insertions, or substitutions of residues of the known amino acid sequence of the isolated polypeptide. The mutation sites may be modified individually or in series, for instance by: (1) substituting first with conserved amino acids and then with more radical choices depending on the results achieved; (2) deleting the target residue; or (3) inserting residues of the same or a different class adjacent to the located site, or combinations of options 1-3.

Clearly, such site-directed methods are one way in which an N-terminal cysteine (or a functional equivalent) can be introduced into a given polypeptide sequence to provide the attachment site for a hydrophobic moiety. Other well-known methods of site-directed mutagenesis are detailed in the below-cited references, which are incorporated by reference herein.

Alanine scanning Mutagenesis: See Cunningham and Wells, (1989) Science 244, 1081-1085).
Oligonucleotide-Mediated Mutagenesis: See, for example, Adelman et al., (1983) DNA 2,183.
Cassette Mutagenesis: See Wells et al., (1985) Gene 34, 315.
Combinatorial Mutagenesis: See, for example, Ladner et al., W0 88/06630
Phage Display Strategies: See, for example the review by Marks et al., J. Biol. Chemistry: 267 16007-16010 (1992).

### F. Other Variants of Alpha 4 Integrin antagonists

Variants can differ from other alpha 4 integrin antagonists in amino acid sequence or in ways that do not involve sequence, or both. The most preferred polypeptides of the invention have preferred non-sequence modifications that include *in vivo* or *in vitro* chemical derivatization (e.g., of their N-terminal end), as well as possible changes in acetylation, methylation, phosphorylation, amidation, carboxylation, or glycosylation.

Other analogs include a protein or its biologically active fragments whose sequences differ from TA2 or those found in U.S. Patents 5,840,299 or U.S. 5,888,507; U.S. 5,932.214 or PCT US/94/00266 by one or more conservative amino acid substitutions or by one or more non conservative amino acid substitutions, or by deletions or insertions which do not abolish the isolated protein's biological activity. Conservative substitutions typically include the substitution of one amino acid for another with similar characteristics such as substitutions within the following groups: valine, alanine and glycine; leucine and isoleucine; aspartic acid and glutamic acid: asparagine and glutamine; serine and threonine; lysine and arginine: and phenylalanine and tyrosine. The non-polar hydrophobic amino acids include alanine, leucine, isoleucine, valine, proline, phenylalanine, tryptophan, and methionine. The polar neutral amino acids include glycine, serine, threonine, cysteine, tyrosine, asparagine, and glutamine. The positively charged (basic) amino acids include arginine, lysine, and histidine. The negatively charged (acidic) amino acids include aspartic acid and glutamic acid. Other conservative substitutions can be readily known by workers of ordinary skill. For example, for the amino acid alanine, a conservative substitution can be taken from any one of D-alanine, glycine, beta-alanine, L-cysteine, and D-cysteine. For lysine, a replacement can be any one of D-lysine, arginine, D-arginine, homo-arginine, methionine, D-methionine, ornithine, or D-ornithine.

Other analogs used within the invention are those with modifications which increase peptide stability. Such analogs may contain, for example, one or more non-peptide bonds (which replace the peptide bonds) in the peptide sequence. Also included are: analogs that include residues other than naturally occurring L-amino acids, such as D-amino acids or non-naturally occurring or synthetic amino acids such as beta or gamma amino acids and cyclic analogs. Incorporation of D- instead of L-amino acids into the isolated hedgehog polypeptide may increase its resistance to proteases. See, U.S. Patent 5,219.990 *supra.*

Preferred antibody homologs include an amino acid sequence at least 60%, 80%, 90%, 95%, 98%, or 99% homologous to an amino acid sequence of TA2 antibody of or sequence at least 60%, 80%, 90%, 95%, 98%, or 99% homologous to an amino acid sequences described in, for instance, U.S. Patent 5,840,299 (e.g, SEQ ID NO 15-light chain variable region; SEQ ID NO: 17- heavy chain variable region); U.S. Patent 5,932,214 (e.g., SEQ ID NOS: 2 and 4): and published patent application W094/16094 (those sequences found in the anti-VLA4 antibody of cell line ATCC CRL 11175).

### G. Polymer Conjugate Forms

Within the broad scope of the present invention, a single polymer molecule may be employed for conjugation with an alpha4 integrin antagonist, although it is also contemplated that more than one polymer molecule can be attached as well. Conjugated alpha4 integrin antagonist compositions of the invention may find utility in both *in vivo* as well as non-in vivo applications. Additionally, it will be recognized that the conjugating polymer may utilize any other groups, moieties, or other conjugated species, as appropriate to the end use application. By way of example, it may be useful in some applications to covalently bond to the polymer a functional moiety imparting UV-degradation resistance, or antioxidation, or other properties or characteristics to the polymer. As a further example, it may be advantageous in some applications to functionalize the polymer to render it reactive and enable it to cross-link to a drug molecule, to enhance various properties or characteristics of the overall conjugated material. Accordingly, the polymer may contain any functionality, repeating groups, linkages, or other constitutent structures which do not preclude the efficacy of the conjugated alpha4 integrin antagonist composition for its intended purpose. Other objectives and advantages of the present invention will be more fully apparent from the ensuing disclosure and appended claims.

Illustrative polymers that may usefully be employed to achieve these desirable characteristics are described herein below in exemplary reaction schemes. In covalently bonded antagonist/polymer conjugates, the polymer may be functionalized and then coupled to free amino acid(s) of the antagonist to form labile bonds.

Alpha4 integrin antagonists are conjugated most preferably via a terminal reactive group on the polymer although conjugations can also be branched from non-terminal reactive groups. The polymer with the reactive group(s) is designated herein as "activated polymer". The reactive group selectively reacts with free amino or other reactive groups on the antagonist molecule. The activated polymer(s) is reacted so that attachment may occur at any available alpha4 integrin antagonist amino group such as the alpha amino groups or the epsilon-amino groups of lysines. Free carboxylic groups, suitably activated carbonyl groups, hydroxyl, guanidyl, oxidized carbohydrate moieties and mercapto groups of the alpha4 integrin antagonist (if available) can also be used as attachment sites.

Although the polymer may be attached anywhere on the alpha4 integrin antagonist molecule, a preferred site for polymer coupling to integrin antagonists (particularly those that are proteins) is the N-terminus of the alpha4 integrin antagonist. Secondary site(s) are at or near the C-terminus and through sugar moieties (if any). Thus, the invention contemplates: (i) N-terminally coupled polymer conjugates of alpha4 integrin antagonists; (ii) C-terminally coupled polymer conjugates of alpha4 integrin antagonists; (iii) sugar-coupled conjugates; (iv) as well as N-, C- and sugar-coupled polymer conjugates of alpha4 integrin antagonists.

Generally from about 1.0 to about 10 moles of activated polymer per mole of antagonist, depending on antagonist concentration, is employed. The final amount is a balance between maximizing the extent of the reaction while minimizing non-specific modifications of the product and, at the same time, defining chemistries that will maintain optimum activity, while at the same time optimizing, if possible, the half-life of the antagonist. Preferably, at least about 50% of the biological activity of the antagonist is retained, and most preferably 100% is retained.

The reactions may take place by any suitable art-recognized method used for reacting biologically active materials with inert polymers. Generally the process involves preparing an activated polymer (that may have at least one terminal hydroxyl group) and thereafter reacting the antagonist with the activated polymer to produce the soluble protein suitable for formulation. The above modification reaction can be performed by several methods, which may involve one or more steps.

As mentioned above, certain embodiments of the invention utilize the N-terminal end of an alpha4 integrin antagonist as the linkage to the polymer. Suitable conventional methods are available to selectively obtain an N-terminally modified alpha4 integrin antagonist. One method is exemplified by a reductive alkylation method which exploits differential reactivity of different types of primary amino groups (the epsilon amino groups on the lysine versus the amino groups on an N-terminal methionine) available for derivatization on a suitable alpha4 integrin antagonist. Under the appropriate selection conditions, substantially selective derivatization of a suitable alpha4 integrin antagonist at an N-terminus thereof with a carbonyl group containing polymer can be achieved. The reaction is performed at a pH which allows one to take advantage of the pKa differences between the epsilon-amino groups of the lysine residues and that of the alpha-amino group of an N-terminal residue of alpha4 integrin antagonist. This type of chemistry is well known to persons with ordinary skill in the art.

A strategy for targeting a polyalkylene glycol polymer such as PEG to the C-terminus of an alpha4 integrin antagonist (e.g., as a protein) would be to chemically attach or genetically engineer a site that can be used to target the polymer moiety. For example, incorporation of a Cys at a site that is at or near the C-terminus of a protein would allow specific modification using art recognized maleimide, vinylsulfone or haloacetate- activated derivatives of polyalkylene glycol (e.g., PEG). These derivatives can be used specifically for modification of the engineered cysteines due to the high selectively of these reagents for Cys. Other strategies such as incorporation of a histidine tag which can be targeted (Fancy et. al., (1996) Chem. & Biol. 3: 551) or an additional glycosylation site on a protein, represent other alternatives for modifying the C-terminus of an alpha4 integrin antagonist.

Methods for targeting sugars as sites for chemical modification are also well known and therefore it is likely that a polyalkylene glycol polymer can be added directly and specifically to sugars (if any) on an alpha4 integrin antagonist that have been activated through oxidation. For example, a polyethyleneglycol-hydrazide can be generated which forms relatively stable hydrazone linkages by condensation with aldehydes and ketones. This property has been used for modification of proteins through oxidized oligosaccharide linkages. See Andresz, H. et al., (1978), Makromol. Chem. 179: 301. In particular, treatment of PEG-carboxymethyl hydrazide with nitrite produces PEG-carboxymethyl azide which is an electrophilically active group reactive toward amino groups. This reaction can be used to prepare polyalkylene glycol-modified proteins as well. See, U.S. Patents 4,101,380 and 4,179,337.

One can use art recognized thiol linker-mediated chemistry to further facilitate cross-linking of proteins to form multivalent alpha 4 integrin antagonist compositions. In particular, one can generate reactive aldehydes on carbohydrate moieties with sodium periodate, forming cystamine conjugates through the aldehydes and inducing cross-linking via the thiol groups on the cystamines. See Pepinsky, B. et al., (1991), J. Biol. Chem., 266: 18244-18249 and Chen, L.L. et al., (1991) J. Biol. Chem., 266: 18237-18243. Therefore, this type of chemistry would also be appropriate for modification with polyalkylene glycol polymers where a linker is incorporated into the sugar and the polyalkylene glycol polymer is attached to the linker. While aminothiol or hydrazine-containing linkers will allow for addition of a single polymer group, the structure of the linker can be varied so that multiple polymers are added and/or that the spatial orientation of the polymer with respect to the alpha4 integrin antagonist is changed.

In the practice of the present invention, polyalkylene glycol residues of C1-C4 alkyl polyalkylene glycols, preferably polyethylene glycol (PEG), or poly(oxy)alkylene glycol residues of such glycols are advantageously incorporated in the polymer systems of interest. Thus, the polymer to which the protein is attached can be a homopolymer of polyethylene glycol (PEG) or is a polyoxyethylated polyol, provided in all cases that the polymer is soluble in water at room temperature. Non-limiting examples of such polymers include polyalkylene oxide homopolymers such as PEG or polypropylene glycols, polyoxyethylenated glycols, copolymers thereof and block copolymers thereof, provided that the water solubility of the block copolymer is maintained. Examples of polyoxyethylated polyols include, for example, polyoxyethylated glycerol, polyoxyethylated sorbitol, polyoxyethylated glucose, or the like. The glycerol backbone of polyoxyethylated glycerol is the same backbone occurring naturally in, for example, animals and humans in mono-, di-, and triglycerides. Therefore, this branching would not necessarily be seen as a foreign agent in the body.

As an alternative to polyalkylene oxides, dextran, polyvinyl pyrrolidones, polyacrylamides, polyvinyl alcohols, carbohydrate-based polymers and the like may be used. Those of ordinary skill in the art will recognize that the foregoing list is merely illustrative and that all polymer materials having the qualities described herein are contemplated.

The polymer need not have any particular molecular weight, but it is preferred that the molecular weight be between about 300 and 100,000, more preferably between 10,000 and 40,000. In particular, sizes of 20,000 or more are best at preventing loss of the product due to filtration in the kidneys.

Polyalkylene glycol derivatization has a number of advantageous properties in the formulation of polymer-alpha4 integrin antagonist conjugates, as associated with the following properties of polyalkylene glycol derivatives: improvement of aqueous solubility, while at the same time eliciting no antigenic or immunogenic response; high degrees of biocompatibility; absence of in vivo biodegradation of the polyalkylene glycol derivatives; and ease of excretion by living organisms.

Moreover, in another aspect of the invention, one can utilize an alpha4 integrin antagonist covalently bonded to the polymer component in which the nature of the conjugation involves cleavable covalent chemical bonds. This allows for control in terms of the time course over which the polymer may be cleaved from the alpha4 integrin antagonist. This covalent bond between the alpha4 integrin antagonist and the polymer may be cleaved by chemical or enzymatic reaction. The polymer-alpha4 integrin antagonist product retains an acceptable amount of activity. Concurrently. portions of polyethylene glycol are present in the conjugating polymer to endow the polymer-alpha4 integrin antagonist conjugate with high aqueous solubility and prolonged blood circulation capability. As a result of these improved characteristics the invention contemplates parenteral, nasal, and oral delivery of both the active polymer-alpha4 integrin antagonist species and, following hydrolytic cleavage, bioavailability of the alpha4 integrin antagonist per se, in in vivo applications.

It is to be understood that the reaction schemes described herein are provided for the purposes of illustration only and are not to be limiting with respect to the reactions and structures which may be utilized in the modification of the alpha4 integrin antagonist, e.g., to achieve solubility, stabilization, and cell membrane affinity for parenteral and oral administration. The activity and stability of the alpha4 integrin antagonist conjugates can be varied in several ways, by using a polymer of different molecular size. Solubilities of the conjugates can be varied by changing the proportion and size of the polyethylene glycol fragment incorporated in the polymer composition.

### III.Therapeutic Applications

The amount of active ingredient that may be combined with the carrier materials to produce a single dosage form will vary depending upon the host treated, and the particular mode of administration. It should be understood, however, that a specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease being treated. The amount of active ingredient may also depend upon the therapeutic or prophylactic agent, if any, with which the ingredient is co-administered.

The dosage and dose rate of the compounds of this invention effective to prevent, suppress or inhibit cell adhesion will depend on a variety of factors, such as the nature of the inhibitor, the size of the patient, the goal of the treatment, the nature of the pathology to be treated, the specific pharmaceutical composition used, and the judgment of the treating physician. Dosage levels of between about 0.001 and about 100 mg/kg body weight per day, preferably between about 0.1 and about 50 mg/kg body weight per day of the active ingredient compound are useful. Most preferably, the VLA-4 binding agent, if an antibody or antibody derivative, will be administered at a dose ranging between about 0. 1 mg/kg body weight/day and about 20 mg/kg bodv weight/day, preferably ranging between about 0.1 mg/kg body weight/day and about 10 mg/kg body weight/day and at intervals of every 1-14 days. For non-antibody or small molecule binding agents, the dose range should preferably be between molar equivalent amounts to these amounts of antibody. Preferably, an antibody composition is administered in an amount effective to provide a plasma level of antibody of at least 1 mg/ml. Optimization of dosages can be determined by administration of the binding agents, followed by assessment of the coating of integrin-positive cells by the agent over time after administered at a given dose in vivo.

The presence of the administered agent may be detected in vitro (or ex vivo) by the inability or decreased ability of the individual's cells to bind the same agent which has been itself labelled (e.g., by a fluorochrome). The preferred dosage should produce detectable coating of the vast majority of integrin-positive cells. Preferably, coating is sustained in the case of an antibody homolog for a 1- 14 day period.

Another preferred modality for introducing the antagonist is through combination therapy with a pharmacological agent. The pharmacological agent is preferably an agent with some degree of therapeutic efficacy in treating acute brain injury. Such agents may include, but are not limited to, thrombolytic agents such as plasminogen or urokinase, agents that target excitotoxic mechanisms such as Selfotel tm or Aptiganel tm, agents that target nitric oxide associated neuronal damage such as Lubeluzole tm, agents that target ischemia associated neuronal cellular membrane damage such as Tirilizad tm, agents that target anti-inflammatory mechanisms such as Enlimomab tm. The agent may be combined with the alpha 4 integrin antagonists of the invention either prior to, during, or after administration of the antagonists.

### IV. Formulations and Methods for Treatment

The method of treatment according to this invention involves administering internally or topically to the subject an effective amount of active compound. Doses of active compounds in the inventive method are an efficacious, non toxic quantity. Persons skilled in the art of using routine clinical testing are able to determine optimum doses for the particular ailment being treated.

Standard tests for neurological recovery (eg. NIH Stroke Scale, Barthel Index, modified Rankin Scale, Glasgow Outcome Scale) will be employed by skilled artisans to determine efficacy. The desired dose is administered to a subject one or more times daily, intravenously, orally, rectally, parenterally, intranasally, topically, or by inhalation. The desired dose may also be given by continuous intravenous infusion.

In parenteral administration of alpha4 integrin inhibitors pursuant to this invention, the compounds may be formulated in aqueous injection solution which may contain antioxidants, buffers, bacteriostats, etc. Extemporaneous injection solutions may be prepared from sterile pills, granules, or tablets which may contain diluents, dispersing and surface active agents, binders and lubricants which materials are all well known to the experienced skilled artisan.

In the case of oral administration, fine powders or granules of the compound may be formulated with diluents and dispersing and surface active agents, and may be prepared in water or in a syrup, in capsules or cachets in the dry state or in a non aqueous suspension where a suspending agent may be included. The compounds may also be administered in tablet form along with optional binders and lubricants, or in a suspension in water or syrup or an oil or in a water/oil emulsion and may include flavoring, preserving, suspending, thickening and emulsifying agents. The granules or tablets for oral administration may be coated or other pharmaceutically acceptable agents and formulations may be utilized which are all known to those skilled in the pharmaceutical art.

Solid to liquid carriers can also be used. Solid carriers include starch, lactose, calcuim, sulfate dihydrate, terra alba, sucrose, talc, gelatin, agar, pectin, acacia, magnesium stearate, and stearic acid, Liquid carriers include syrup, peanut oil, olive oil, saline and water. Ointments and creams are prepared using known polymeric materials such as various acrylic-based polymers selected to provide desired release characteristics. Suppositories are prepared from standard bases such as polyethylene glycol and cocoa butter.

The methods of treatment provided by the present invention relate to methods for treating injuries to the CNS in a patient, comprising administering an alpha4 integrin. In other embodiments, the methods further include the administration of a pharmacological agent to the patient. In preferred embodiments, the pharmacological agent is a thrombolytic agent, a neuroprotective agent, an anti-inflammatory agent, a steroid, a cytokine or a growth factor. The thrombolytic agent used in the present invention is preferably tissue plasminogen activator or urokinase. The neuroprotective agent used in the present invention is preferably an agonist to a receptor selected from the group consisting of: N-Methyl-D aspartate receptor (NMDA), α-amino-3-hydroxy-5-methyl-4-isoxazoleproprionic acid receptor (AMPA), glycine receptor, calcium channel receptor, bradykinin B2 receptor and sodium channel receptor, or from the group consisting of: the bradykinin B1 receptor, α-amino butyric acid (GABA) receptor, and Adenosine A1 receptor. Anti-inflammatory agents for use in the present invention include interleukin-1 and tumor necrosis factor family members.

As contemplated by the present invention, the apha4 integrin antagonist used in the methods of treatment may be an antibody homolog, and preferably a humanized antibody homolog or a fragment of an antibody homolog. In other embodiments, the antibody homolg may be linked to a polymer molecule. In the methods of the present invention, the alpha4 integrin antagonist may alternatively be capable of antagonizing a single alpha4 subunit-containing integrin, or more than one alpha4 subunit-containing integrin.

### EXAMPLES

### Example 1 : Protocol for Reversible Middle Cerebral Artery Occlusion in the rat.

Male Sprague Dawley (SD) or spontaneously hypertensive rats (SHRS) were anesthetized using isoflurane and the right middle cerebral artery (MCAO) occluded by insertion of a 4-0 nylon monofilament up the internal carotid artery to the origin of the middle cerebral artery (MCA) (Zea Longa et al, 1989 Stroke 20:84). After 1h the filament was retracted, the ischemic territory reperfused and the animal allowed to recover. After 24h the rats were sacrificed, at which time brains were removed and analyzed histologically to quantify infarct volume.

Groups of animals were treated with either vehicle (PBS) or the bradykinin B₂ receptor antagonist Hoe 140 (Hoechst) by continuous subcutaneous infusion via osmotic mini-pumps. Primed mini osmotic pumps (Alza Corp.,) were implanted into the subcutaneous space at the scruff of the neck immediately prior to induction of cerebral ischemia. The pumps were loaded to release 300ng/kg/min Hoe 140 and delivered compound or vehicle at a rate of 8µl/h.

In a separate experiment groups of animals were treated with either vehicle (sterile isotonic saline), TA2 (mouse anti rat VLA4: Seikagaku America Inc.) or an isotype control antibody (mouse anti-human LFA3: obtained from Biogen, Inc.). All treatments were administered 24h before surgery intravenously (2.5mg/kg or appropriate volume of vehicle)

### Example 2: Results of Reversible Middle Cerebral Artery Occlusion Model

Vehicle treated control rats that underwent MCAO sustained extensive lesions throughout cortical and subcortical regions of the brain. The ischemic hemisphere was markedly swollen and significant behavioral deficits were observed (eg. hemiparesis resulting in rotation and limb weakness). Spontaneously hypertensive rats sustained more extensive and reproducible brain infarcts than Sprague Dawley rats subjected to the same surgical procedure. Infarct volumes are expressed as mean values +/- s.e.m. Statistical analysis was performed using an unpaired Students' t-test (* denotes p<0.05. ** denotes p<0. 01).

Treatment with the bradykinin B₂ receptor antagonist Hoe 140 (n=9) significantly reduced total, cortical and subcortical infarct volume, by 37%, 43% and 17% respectively, compared to vehicle treated controls (n=8) measured 24h after induction of cerebral ischemia in SHRs. In SD rats treatment with the same dose of Hoe 140 (n=6) reduced total, cortical and subcortical infarct volume, by 57%. 93% and 24% respectively, compared to vehicle treated controls (n=7) measured 24h after the induction of cerebral ischemia. These data are consistent with previous findings (Relton et. al, 1997 Stroke 28:1430) and were undertaken as a positive control.

In SHRs pre-treatment with the anti α4 antibody, TA-2 (2.5mg/kg iv, n=10), 24h prior to induction of cerebral ischemia significantly reduced total, cortical and subcortical infarct volumes, by 43%, 47% and 33% respectively, compared animals treated with the same dose of an isotype control antibody (n=15) measured 24h after induction of cerebal ischemia. In SD rats using the same protocol, total, cortical and subcortical infarct volume was significantly reduced by 64%, 65% and 38% respectively.

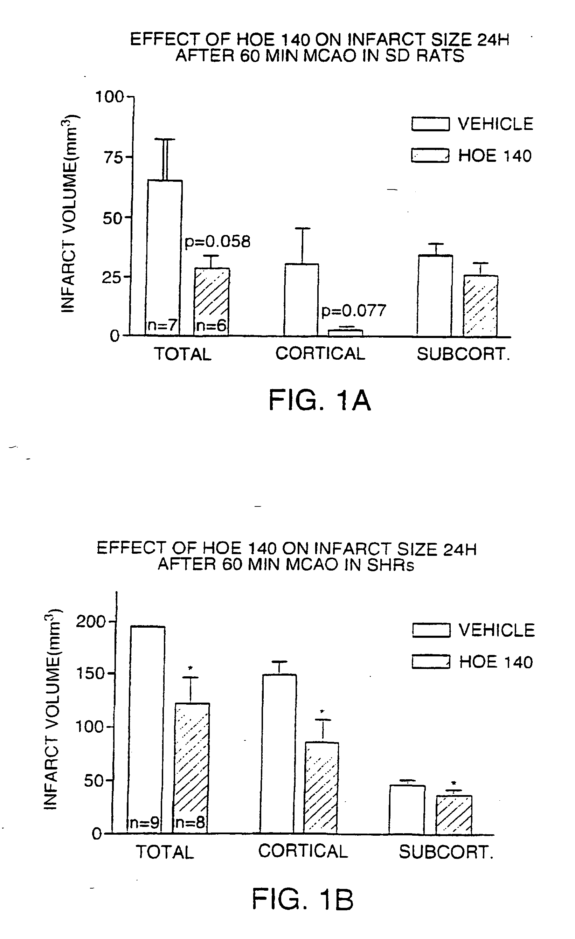

The graphs in Figures 1A and 1B show the effect of hoe 140 on infarct size 24 hours after 60 minute MCAO in Sprague Dawley and spontaneously hypertensive rats. The figures show inhibition of brain infarction following treatment with hoe 140 (300ng/kg/min) by continuous subcutaneous infusion compared to vehicle treated control animals. Infarct size is reduced in cortical and subcortical regions of the brain in both strains of rats.

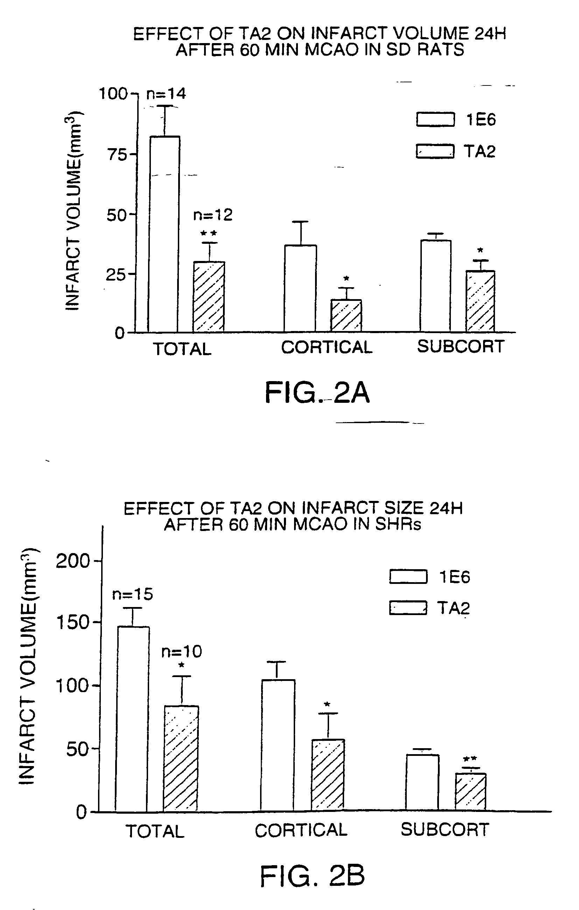

The graphs shown in Figure 2A and 2B show the effect of anti rat alpha4 antibody (TA-2, 2.5mg/kg) on infarct size 24 hours after 60 minute MCAO in Sprague Dawley and spontaneously hypertensive rats. The figure shows significant inhibition of brain infarction following intravenous pre-treatment with TA-2 antibody compared to animals treated with an isotype control antibody. Protection against brain damage was observed in both strains of rats.

These data demonstrate the protective effect of inhibiton of α4 integrins in a model of reversible focal cerebral ischemia in the rat. The pathology of this model is clinically representative of the human condition of stroke and the present data suggest that inhibitors of alpha4 subunit containing integrins may be of significant benefit in the treatment of this and other ischemia-related disorders.

## Claims

1. Use of an anti-α4 integrin antibody or an α4 integrin-binding fragment thereof for the preparation of a pharmaceutical composition for the prophylactic treatment of secondary injuries associated with traumatic brain injury, wherein the secondary injuries are ischemic.

2. An anti-α4 integrin antibody or an α4 intecrin-binding fragment thereof for use in the prophylactic treatment of secondary injuries associated with traumatic brain injury, wherein the secondary injuries are ischemic.

3. The use of claim 1 or the anti-α4 integrin antibody or α4 integrin-binding fragment of claim 2, wherein the pharmaceutical composition further comprises a pharmacological agent.

4. The use or the anti-α4 integrin antibody or α4 integrin-binding fragment of claim 3, wherein the agent is selected from the group consisting of: a neuroprotective agent, an anti-inflammatory agent, a steroid, a cytokine, a growth factor or a thrombolytic agent.

5. The use or the anti-α4 integrin antibody or α4 integrin-binding fragment of claim 4, wherein said neuroprotective agent is
(i) an antagonist of a receptor selected from the group consisting of: an N-Methyl-D aspartate receptor (NMDA), an α-amino-3-hydroxy-5-methyl-4-isoxazoleproprionic acid receptor (AMPA), a glycine receptor, a calcium channel receptor, a bradykinin B2 receptor and a sodium channel receptor; or
(ii) an agonist of a receptor selected from the group consisting of: a bradykinin B 1 receptor, γ-aminobutyric acid (GABA) receptor and an adenosine A1 receptor.

6. The use or the anti-α4 integrin antibody or α4 integrin-binding fragment of claim 4, wherein said anti-inflammatory agent is selected from the group consisting of: an interleukin-1 and a tumor necrosis factor family member.

7. The use or the anti-α4 integrin antibody or α4 integrin-binding fragment of claim 4, wherein said thrombolytic agent is selected from the group consisting of a tissue plasminogen activator and a urokinase.

8. Use of an anti-α4 integrin antibody or an α4 integrin-binding fragment thereof for the preparation of a pharmaceutical composition for the prevention of secondary brain damage resulting from an ischemic insult.

9. An anti-α4 integrin antibody or an α4 integrin-binding fragment thereof for use in the prevention of secondary brain damage resulting from an ischemic insult.

10. The use of claim 8 or the anti-α4 integrin antibody or α4 integrin-binding fragment of claim 9, wherein the secondary brain damage is caused by a hemorrhagic transformation or a cerebral vasospasm.

11. The use of any one of claims 1, 3 to 8 or 10 or the anti-α4 integrin antibody or α4 integrin-binding fragment of any one of claims 2 to 7 or 9 to 10 wherein the anti-α4 integrin antibody or an α4 integrin-binding fragment thereof is selected from the group consisting of:
(i) a monoclonal anti- α4 integrin antibody or an α4 integrin-binding fragment thereof;
(ii) a humanized monoclonal anti- α4 integrin antibody or an α4 integrin-binding fragment thereof;
(iii) a human monoclonal anti- α4 integrin antibody or an α4 integrin-binding fragment thereof;
(iv) a chimeric anti- α4 integrin antibody or an α4 integrin-binding fragment thereof; and
(v) a B epitope-specific anti- α4 integrin-antibody or an α4 integrin-binding fragment thereof.

12. The use of any one of claims 1, 3 to 8, 10 or 11 or the anti-α4 integrin antibody or α4 integrin-binding fragment of any one of claims 2 to 7 or 9 to 11, wherein the anti-α4 integrin-binding fragment thereof is linked to a polymer molecule.

13. The use of any one of claims 1, 3 to 8, or 10 to 12 or the anti-α4 integrin antibody or α4 integrin-binding fragment of any one of claims 2 to 7 or 9 to 12, wherein the anti-α4 integrin antibody or an α4 integrin-binding fragment thereof is capable of antagonizing a single α4 subunit-containing integrin or more than one α4 subunit-containing integrin.

14. The use or the anti-α4 integrin antibody or α4 integrin-binding fragment of claim 13, wherein the anti-α4 integrin antibody or an α4 integrin-binding fragment thereof is capable of antagonizing VLA-4 and α4β7.

15. The use of any one of claims 1, 3 to 8, or 10 to 14 or of the anti-α4 integrin antibody or α4 integrin-binding fragment any one of claims 2 to 7 or 9 to 14, wherein the anti-α4 integrin antibody or an α4 integrin-binding fragment thereof is a human B epitope-specific anti-α4 integrin-antibody or an α4 integrin-binding fragment thereof.

16. The use the anti-α4 integrin antibody or α4 integrin-binding fragment of any one of claims 11 to 15, wherein the anti-α4 integrin antibody or an α4 integrin-binding fragment thereof is a Fab fragment, a Fab' fragment, a F(ab')₂ fragment, or an Fv fragment.

17. The use of claim 1 or the anti-α4 integrin antibody or α4 integrin-binding fragment of claim 2, wherein the secondary injuries comprise hemorrhagic transformations or cerebral vasospasms.

## Patentansprüche

1. Verwendung eines Anti-α4-Integrin-Antikörpers oder eines α4-Integrin-bindenden Fragments davon zur Herstellung einer pharmazeutischen Zusammensetzung zur prophylaktischen Behandlung von sekundären Verletzungen, die mit traumatischer Gehirnverletzung verbunden sind, wobei die sekundären Verletzungen ischämisch sind.

2. Anti-α4-Integrin-Antikörper oder ein α4-Integrin-bindendes Fragment davon zur Verwendung in der prophylaktischen Behandlung von sekundären Verletzungen, die mit traumatischer Gehirnverletzung verbunden sind, wobei die sekundären Verletzungen ischämisch sind.

3. Verwendung nach Anspruch 1 oder der Anti-α4-Integrin-Antikörper oder das α4-Integrin-bindende Fragment nach Anspruch 2, wobei die pharmazeutische Zusammensetzung ferner ein pharmakologisches Mittel umfasst.

4. Verwendung oder der Anti-α4-Integrin-Antikörper oder das α4-Integrin-bindende Fragment nach Anspruch 3, wobei das Mittel ausgewählt ist aus der Gruppe bestehend aus: einem neuroprotektiven Mittel, einem entzündungshemmenden Mittel, einem Steroid, einem Cytokin, einem Wachstumsfaktor oder einem thrombolytischen Mittel.

5. Verwendung oder der Anti-α4-Integrin-Antikörper oder das α4-Integrin-bindende Fragment nach Anspruch 4, wobei das neuroprotektive Mittel Folgendes ist:
(i) ein Antagonist eines Rezeptors ausgewählt aus der Gruppe bestehend aus: einem N-Methyl-D-aspartat-Rezeptor (NMDA), einem α-Amino-3-hydroxy-5-methyl-4-isoxazolproprionsäure-Rezeptor (AMPA), einem Glycin-Rezeptor, einem Calcium-Kanal-Rezeptor, einem Bradykinin-B2-Rezeptor und einem Natrium-Kanal-Rezeptor; oder
(ii) einem Agonist eines Rezeptors ausgewählt aus der Gruppe bestehend aus: einem Bradykinin B1-Rezeptor, γ-Aminobuttersäure (GABA)-Rezeptor und einem Adenosin-A1-Rezeptor.

6. Verwendung oder der Anti-α4-Integrin-Antikörper oder das α4-Integrin-bindende Fragment nach Anspruch 4, wobei das entzündungshemmende Mittel ausgewählt ist aus der Gruppe bestehend aus: einem Interleukin-1 und einem Tumornekrosefaktor-Familienmitglied.

7. Verwendung oder der Anti-α4-Integrin-Antikörper oder das α4-Integrin-bindende Fragment nach Anspruch 4, wobei das thrombolytische Mittel ausgewählt ist aus der Gruppe bestehend aus einem Gewebe-Plasminogen-Aktivator und einer Urokinase.

8. Verwendung eines Anti-α4-Integrin-Antikörpers oder ein α4-Integrin-bindendes Fragment davon zur Herstellung einer pharmazeutischen Zusammensetzung zur Prävention von sekundären Hirnschäden infolge eines ischämischen Insults.

9. Anti-α4-Integrin-Antikörper oder ein α4-Integrin-bindendes Fragment davon zur Verwendung in der Prävention von sekundären Hirnschäden infolge eines ischämischen Insults.

10. Verwendung von Anspruch 8 oder der Anti-α4-Integrin-Antikörper oder das α4-Integrin-bindende Fragment nach Anspruch 9, wobei die sekundären Hirnschäden durch eine hämorrhagische Transformation oder einen zerebralen Vasospasmus verursacht wurden.

11. Verwendung nach einem der Ansprüche 1, 3 bis 8 oder 10 oder der Anti-α4-Integrin-Antikörper oder das α4-Integrin-bindende Fragment nach einem der Ansprüche 2 bis 7 oder 9 bis 10, wobei der Anti-α4-Integrin-Antikörper oder ein α4-Integrin-bindendes Fragment davon ausgewählt ist aus der Gruppe bestehend aus:
(i) einem monoklonalen Anti-α4-Integrin-Antikörper oder einem α4-Integrin-bindenden Fragment davon;
(ii) einem humanisierten monoklonalen Anti-α4-Integrin-Antikörper oder einem α4-Integrin-bindenden Fragment davon;
(iii) einem humanen monoklonalen Anti-α4-Integrin-Antikörper oder einem α4-Integrin-bindenden Fragment davon;
(iv) einem chimären Anti-α4-Integrin-Antikörper oder einem α4-Integrin-bindenden Fragment davon; und
(v) einem B-Epitop-spezifischen Anti-α4-Integrin-Antikörper oder einem α4-Integrin-bindenden Fragment davon.

12. Verwendung nach einem der Ansprüche 1, 3 bis 8, 10 oder 11 oder der Anti-α4-Integrin-Antikörper oder das α4-Integrin-bindende Fragment nach einem der Ansprüche 2 bis 7 oder 9 bis 11, wobei das Anti-α4-Integrin-bindende Fragment davon an ein Polymermolekül gebunden ist.

13. Verwendung nach einem der Ansprüche 1, 3 bis 8 oder 10 bis 12 oder der Anti-α4-Integrin-Antikörper oder das α4-Integrin-bindende Fragment nach einem der Ansprüche 2 bis 7 oder 9 bis 12, wobei der Anti-α4-Integrin-Antikörper oder ein α4-Integrin-bindendes Fragment davon fähig ist, ein einzelnes die α4-Untereinheit enthaltendes Integrin oder mehr als ein die α4-Untereinheit enthaltendes **Integrin** zu antagonisieren.

14. Verwendung oder der Anti-α4-Integrin-Antikörper oder das α4-Integrin-bindende Fragment nach Anspruch 13, wobei der Anti-α4-Integrin-Antikörper oder ein α4-Integrin-bindendes Fragment davon fähig ist, VLA-4 und α4β7 zu antagonisieren.

15. Verwendung nach einem der Ansprüche 1, 3 bis 8 oder 10 bis 14 oder der Anti-α4-Integrin-Antikörper oder das α4-Integrin-bindende Fragment nach einem der Ansprüche 2 bis 7 oder 9 bis 14, wobei der Anti-α4-Integrin-Antikörper oder ein α4-Integrin-bindendes Fragment davon ein menschlicher B-Epitop-spezifischer Anti-α4-Integrin-Antikörper oder ein α4-Integrin-bindendes Fragment davon ist.

16. Verwendung des Anti-α4-Integrin-Antikörpers oder des α4-Integrin-bindenden Fragments nach einem der Ansprüche 11 bis 15, wobei der Anti-α4-Integrin-Antikörper oder ein α4-Integrin-bindendes Fragment davon ein Fab-Fragment, ein Fab'-Fragment, ein F(ab')₂-Fragment oder ein Fv-Fragment ist.

17. Verwendung nach Anspruch 1 oder der Anti-α4-Integrin-Antikörper oder das α4-Integrin-bindende Fragment nach Anspruch 2, wobei die sekundären Verletzungen hämorrhagische Transformationen oder zerebrale Vasospasmen umfassen.

## Revendications

1. Utilisation d'un anticorps anti-intégrine α4 ou d'un fragment de liaison à l'intégrine α4 de celui-ci pour la préparation d'une composition pharmaceutique destinée au traitement prophylactique de blessures secondaires associées à une blessure traumatique du cerveau, dans laquelle les blessures secondaires sont ischémiques.

2. Anticorps anti-intégrine α4 ou d'un fragment de liaison à l'intégrine α4 de celui-ci destiné à être utilisé dans le traitement prophylactique de blessures secondaires associées à une blessure traumatique du cerveau, dans laquelle les blessures secondaires sont ischémiques.

3. Utilisation selon la revendication 1 ou anticorps anti-intégrine α4 ou fragment de liaison à l'intégrine α4 selon la revendication 2, dans laquelle la composition pharmaceutique comprend en outre un agent pharmacologique.

4. Utilisation ou anticorps anti-intégrine α4 ou fragment de liaison à l'intégrine α4 selon la revendication 3, dans laquelle l'agent est choisi dans l'ensemble constitué d'un agent neuroprotecteur, d'un agent anti-inflammatoire, d'un stéroïde, d'une cytokine, d'un facteur de croissance ou d'un agent thrombolytique.

5. Utilisation ou anticorps anti-intégrine α4 ou fragment de liaison à l'intégrine α4 selon la revendication 4, dans laquelle ledit agent neuroprotecteur est
(i) un antagoniste d'un récepteur choisi dans l'ensemble constitué d'un récepteur de N-méthyl-D-aspartate (NMDA), d'un récepteur d'acide α-amino-3-hydroxy-5-méthyl-4-isoxazolepropionique (AMPA), d'un récepteur de la glycine, d'un récepteur de canal calcique, d'un récepteur de bradykinine B2 et d'un récepteur de canal sodique ; ou
(ii) un agoniste d'un récepteur choisi dans l'ensemble constitué d'un récepteur de bradykine B1, d'un récepteur d'acide γ-aminobutyrique (GABA) et d'un récepteur d'adénosine A1.

6. Utilisation ou anticorps anti-intégrine α4 ou fragment de liaison à l'intégrine α4 selon la revendication 4, dans laquelle ledit agent anti-inflammatoire est choisi dans l'ensemble constitué d'une interleukine-1 et d'un membre de la famille des facteurs de nécrose tumorale.

7. Utilisation ou anticorps anti-intégrine α4 ou fragment de liaison à l'intégrine α4 selon la revendication 4, dans laquelle ledit agent thrombolytique est choisi dans l'ensemble constitué d'un activateur tissulaire du plasminogène et d'une urokinase.

8. Utilisation d'un anticorps anti-intégrine α4 ou d'un fragment de liaison à l'intégrine α4 de celui-ci pour la préparation d'une composition pharmaceutique destinée à la prévention de dommages secondaires du cerveau dus à un accident ischémique.

9. Anticorps anti-intégrine α4 ou un fragment de liaison à l'intégrine α4 de celui-ci, destiné à un emploi dans la prévention de dommages secondaires du cerveau dus à un accident ischémique.

10. Utilisation selon la revendication 8 ou un anticorps anti-intégrine α4 ou un fragment de liaison à l'intégrine α4 selon la revendication 9, dans laquelle le dommage secondaire du cerveau est causé par une transformation hémorragique ou un angiospasme cérébral.

11. Utilisation selon l'une quelconque des revendications 1, 3 à 8 ou 10 ou anticorps anti-intégrine α4 ou fragment de liaison à l'intégrine α4 selon l'une des revendications 2 à 7 ou 9 à 10, dans lequel l'anticorps anti-intégrine α4 ou un fragment de liaison à l'intégrine α4 de celui-ci est choisi dans l'ensemble constitué de :
(i) un anticorps anti-intégrine α4 monoclonal ou un fragment de liaison à l'intégrine α4 de celui-ci ;
(ii) un anticorps anti-intégrine α4 monoclonal humanisé ou un fragment de liaison à l'intégrine α4 de celui-ci ;
(iii) un anticorps anti-intégrine α4 monoclonal humain ou un fragment de liaison à l'intégrine α4 de celui-ci ;
(iv) un anticorps anti-intégrine α4 chimérique ou un fragment de liaison à l'intégrine α4 de celui-ci ; et
(v) un anticorps anti-intégrine α4 spécifique de l'épitope B ou un fragment de liaison à l'intégrine α4 de celui-ci ;

12. Utilisation selon l'une quelconque des revendications 1, 3 à 8, 10 ou 11 ou anticorps d'intégrine anti-α4 ou fragment de liaison à l'intégrine α4 selon l'une des revendications 2 à 7 ou 9 à 11, dans lequel le fragment de liaison anti-intégrine α4 de celui-ci est lié à une molécule polymère.

13. Utilisation selon l'une quelconque des revendications 1,3 à 8 ou 10 à 12 ou anticorps anti-intégrine α4 ou fragment de liaison à l'intégrine α4 selon l'une quelconque des revendications 2 à 7 ou 9 à 12, dans lequel l'anticorps anti-intégrine α4 ou un fragment de liaison à l'intégrine α4 de celui-ci est capable d'avoir une action antagoniste contre une intégrine contenant une seule sous-unité α4 ou une intégrine contenant plus d'une sous-unité α4.

14. Utilisation ou anticorps anti-intégrine α4 ou fragment de liaison à l'intégrine α4 selon la revendication 13, dans lequel l'anticorps d'intégrine anti-α4 ou un fragment de liaison à l'intégrine α4 de celui-ci est capable d'avoir une action antagoniste contre VLA-4 et α4β7.

15. Utilisation selon l'une quelconque des revendications 1, 3 à 8 ou 10 à 14 ou anticorps anti-intégrine α4 ou fragment de liaison à l'intégrine α4 selon l'une quelconque des revendications 2 à 7 ou 9 à 14, dans lequel l'anticorps d'intégrine anti-α4 ou un fragment de liaison à l'intégrine α4 de celui-ci est un anticorps anti-intégrine α4 spécifique d'un épitope B humain ou un fragment de liaison à l'intégrine α4 de celui-ci.

16. Utilisation de l'anticorps anti-intégrine α4 ou fragment de liaison à l'intégrine α4 selon l'une quelconque des revendications 11 à 15, dans lequel l'anticorps anti-intégrine α4 ou un fragment de liaison à l'intégrine α4 de celui-ci est un fragment Fab, un fragment Fab', un fragment F(ab')₂ ou un fragment Fv.

17. Utilisation selon la revendication 1 ou anticorps anti-intégrine α4 ou fragment de liaison à l'intégrine α4 selon la revendication 2, dans lequel les blessures secondaires comprennent des transformations hémorragiques ou des angiospasmes cérébraux.
